(19) Europäisches Patentamt
European Patent Office
Office européen des brevets

(11) EP 1 279 671 B1

(12) # EUROPÄISCHE PATENTSCHRIFT

(45) Veröffentlichungstag und Bekanntmachung des Hinweises auf die Patenterteilung:
**16.02.2005 Patentblatt 2005/07**

(51) Int Cl.[7]: **C07D 301/12**, C07D 303/32, C07D 303/36, C07D 303/48, C07D 405/04

(21) Anmeldenummer: **02015587.5**

(22) Anmeldetag: **15.07.2002**

(54) **Polyaminosäure-katalysiertes Verfahren zur enantioselektiven Epoxidierung von alpha,beta-ungesättigten Enonen und alpha,beta-ungesättigten-Sulfonen**

Process for enantioselective epoxidation of alpha, beta-unsaturated enones and alpha, beta-unsaturated sulfones catalysed by polyamino acids

Procédé pour l'époxydation enantiosélective d'énones alpha, beta-insaturées et de sulfones alpha, beta-insaturées, catalysé par des acides polyaminés

(84) Benannte Vertragsstaaten:
**AT BE BG CH CY CZ DE DK EE ES FI FR GB GR IE IT LI LU MC NL PT SE SK TR**

(30) Priorität: **27.07.2001 DE 10136131**

(43) Veröffentlichungstag der Anmeldung:
**29.01.2003 Patentblatt 2003/05**

(73) Patentinhaber: **Bayer Chemicals AG**
**51368 Leverkusen (DE)**

(72) Erfinder:
- **Geller, Thomas, Dr.**
  **51373 Leverkusen (DE)**
- **Krüger, Christa Maria, Dr.**
  **48149 Münster (DE)**
- **Militzer, Hans-Christian, Dr.**
  **51519 Odenthal (DE)**

(56) Entgegenhaltungen:
**EP-A- 0 403 252** **EP-A- 1 006 111**
**EP-A- 1 006 127** **WO-A-96/33183**

- **ADGER B.M. ET AL: "Improved procedure for Julia-Colonna asymmetric epoxidation of alpha, beta-unsaturated ketones. " JOURNAL OF THE CHEMICAL SOCIETY, PERKIN TRANSACTIONS 1., Nr. 23, - 7. Dezember 1997 (1997-12-07) Seiten 3501-3507, XP002219130 CHEMICAL SOCIETY. LETCHWORTH., GB ISSN: 0300-922X**
- **BAURES P.W. ET AL: "An efficient asymmetric synthesis of substituted phenyl glycidic esters" TETRAHEDRON LETTERS., Bd. 31, Nr. 45, 1990, Seiten 6501-6504, XP002006755 ELSEVIER SCIENCE PUBLISHERS, AMSTERDAM., NL ISSN: 0040-4039**
- **JULIA S. ET AL: "Synthetic enzymes. Part 2. Catalytic asymmetric epoxidation by means of polyamino-acids in a triphase system" JOURNAL OF THE CHEMICAL SOCIETY, PERKIN TRANSACTIONS 1., Nr. 6, 1982, Seiten 1317-1324, XP002006756 CHEMICAL SOCIETY. LETCHWORTH., GB ISSN: 0300-922X**
- **DHANDA, ANUPMA ET AL: "PaaSiCats: Novel polyamino acid catalysts" CHIRALITY (2000), 12(5/6), 313-317 , XP008009815**
- **FLOOD R.W. ET AL: "Efficient asymmetric epoxidation of alpha, beta-unsaturated ketones using a soluble triblock polyethyleneglycol-polyamino acid catalyst" ORGANIC LETTERS., Bd. 3, Nr. 5, 8. März 2001 (2001-03-08), Seiten 683-686, XP002219131 ACS, WASHINGTON, DC., US ISSN: 1523-7060**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 279 671 B1

Printed by Jouve, 75001 PARIS (FR)

## Beschreibung

Die Erfindung betrifft ein neues Polyaminosäure-katalysiertes Verfahren zur enantioselektiven Epoxidierung von α,β-ungesättigten Enonen und α,β-ungesättigten Sulfonen in Gegenwart spezieller Co-Katalysatoren.

Chiral, nicht-racemische Epoxide sind als wertvolle Bausteine für die Herstellung von optisch aktiven Wirkstoffen und Materialien bekannt (z.B. a) *Bioorg. Med. Chem.*, **1999,** *7*, 2145-2156; b) *Tetrahedron Lett.*, **1999,** *40*, 5421-5424). Diese Epoxide können durch enantioselektive Epoxidierung von Doppelbindungen hergestellt werden. Hierbei werden zwei Stereozentren in einem synthetischen Schritt aufgebaut. Es ist daher nicht überraschend, dass eine Vielzahl von Methoden entwickelt wurde, um Doppelbindungen enantioselektiv zu epoxidieren. Für neue, verbesserte Methoden zur enantioselektiven Epoxidierung besteht allerdings weiterhin ein großer Bedarf.

Unter den Epoxidierungsmethoden, die jeweils auf spezielle Substrate limitiert sind, finden sich auch Methoden zur enantioselektiven Epoxidierung von α,β-ungesättigten Enonen.

So wurde beispielsweise der Einsatz von chiral, nicht-racemischen Phasentransferkatalysatoren auf Alkaloidbasis zur Epoxidierung von Enonen in *Tetrahedron Lett.,* **1998,** *39*, 7563-7566, *Tetrahedron Lett.*, **1998,** *39*, 1599-1602 und *Tetrahedron Lett.,* **1976,** *21*, 1831-1834 beschrieben.

In *Tetrahedron Lett.*, **1998,** *39*, 7353-7356, *Tetrahedron Lett.*, **1998,** *39*, 7321-7322 und *Angew. Chem., Int. Ed. Engl.,* **1997,** *36*, 410-412 werden ferner Möglichkeiten zur metallkatalysierten asymmetrischen Epoxidierung von Enonen mittels organischer Hydroperoxide beschrieben.

In WO-A-99/52886 wird beschrieben, dass eine enantioselektive Epoxidierung von Enonen in Gegenwart von Katalysatoren möglich ist, die auf Zuckern basieren. Eine weitere Methode zur Epoxidierung unter Verwendung von Zn-Organylen und Sauerstoff in Gegenwart eines Ephedrin-Derivats wurde in *Liebigs Ann./Recueil*, **1997,** 1101-1113 veröffentlicht.

In *Angew. Chem., Int. Ed. Engl.*, **1980,** *19*, 929-930, *Tetrahedron*, **1984,** *40*, 5207-5211 und *J. Chem. Soc., Perkin Trans. 1*, **1982,** 1317-24 wird die Epoxidierungsmethode von Juliá beschrieben, wonach enantiomeren- und diastereomerenangereicherte Polyaminosäuren in Gegenwart von wässriger Wasserstoffperoxid- und NaOH-Lösung sowie eines Aromaten bzw. halogenierten Kohlenwasserstoffs als Lösungsmittel in der Lage sind, die enantioselektive Epoxidierung von α,β-ungesättigten Enonen zu katalysieren. Weiterentwicklungen dieser sogenannten 3-phasigen-Bedingungen finden sich in *Org. Synth.; Mod. Trends, Proc. IUPAC Symp. 6th.,* **1986,** 275. Die Methode wird heute allgemein als Juliá-Colonna-Epoxidierung bezeichnet.

Gemäß EP-A-0 403 252 ist es möglich, bei dieser Juliä-Colonna-Epoxidierung an Stelle der ursprünglichen Lösungsmittel vorteilhafterweise auch aliphatische Kohlenwasserstoffe einzusetzen.

In WO-A-96/33183 wird als spezielle Ausführungsform beschrieben, dass die enantioselektive Epoxidierung von Enonen auch in Gegenwart des Phasentransferkatalysators Aliquat® 336 ($[(CH_3)(C_8H_{17})_3N^+]Cl^-$) durchgeführt werden kann, wenn gleichzeitig eine Polyaminosäure, ein organisches Lösungsmittel wie z.B. Dichlormethan, in Wasser schwerlösliches Natriumperborat als Oxidationsmittel sowie Alkali (z.B. NaOH) zugegen sind.

Trotz dieser Verbesserungen haben die 3-phasigen-Bedingungen deutliche Nachteile. Die Reaktionszeiten liegen nach den Originalbedingungen selbst für reaktive Substrate im Bereich von Tagen. Für die Epoxidierung von *trans*-Chalkon werden in Abhängigkeit der verwendeten Polyaminosäure beispielsweise 1 - 6 Tage benötigt (*Tetrahedron*, **1984,** *40,* 5207-5211). Eine im Reaktionsgefäß ausgeführte Voraktivierung der Polyaminosäure, indem man für 12-48 h im Lösungsmittel unter Zusatz von NaOH-Lösung rührt, verkürzt die Reaktionszeit vieler Substrate auf 1 - 3 Tage. Hierbei ist keine Zwischenaufarbeitung des Katalysators erforderlich (EP-A-0 403 252). In Gegenwart des Systems NaOH/Wasserstoffperoxid kann die Voraktivierungszeit auf minimal 6 h verringert werden (*J. Chem. Soc., Perkin Trans. 1,* **1995,** 1467-1468)

Trotz dieser Verbesserung kann die dreiphasige Methode nicht auf Substrate angewandt werden, welche gegen Hydroxid-Ionen empfindlich sind (*J. Chem. Soc., Perkin Trans. 1*, **1997,** 3501-3507). Ein weiterer Nachteil dieser klassischen Bedingungen besteht darin, dass die Polyaminosäure während der Reaktion (bzw. bereits während der Voraktivierung) ein Gel bildet. Dies schränkt die erwünschte Durchmischung während der Reaktion ein und erschwert die Aufarbeitung des Reaktionsgemisches.

Aus *Tetrahedron Lett.*, **2001,** 42, 3741-43 ist es bekannt, dass unter den 3-phasigen-Bedingungen der Zusatz des Phasentransferkatalysators Aliquat® 336 bei der Epoxidierung des Phenyl-*E*-Styrylsulfons nur zu einer geringen Reaktionsgeschwindigkeit (Reaktionszeit: 4 Tage) und einem schlechtem Enantiomerenüberschuss (21% ee) führt. Es ist bisher kein Beispiel für die Verwendung von Phasentransferkatalyatoren (PTC) zur Epoxidierung von α,β-ungesättigten Enonen unter den klassischen 3-phasigen Juliá-Colonna-Bedingungen bekannt.

Die Juliä-Colonna-Epoxidierung wurde durch eine Änderung der Reaktionsführung weiter verbessert. Gemäß *Chem. Commun.*, **1997,** 739-740 können durch Verwendung von THF, 1,2 Dimethoxyethan, *tert.*-Butylmethylether oder Ethylacetat als Lösungsmittel, einer nicht-nucleophilen Base (z.B. DBU) und des Harnstoff-Wasserstoffperoxid-Komplexes als Oxidationsmittel (pseudo)-wasserfreie Reaktionsbedingurigen verwirklicht werden. Unter diesen sogenannten 2-phasigen Reaktionsbedingungen erfolgt die Epoxidierung deutlich schneller. Auf diesem Wege ist daher gemäß

*J. Chem. Soc., Perkin Trans. 1*, **1997,** 3501-3507 erstmals auch die enantioselektive Epoxidierung von Hydroxid-empfindlichen Enonen unter den Juliä-Colonna-Bedingungen möglich.

Als deutlicher Nachteil erweist sich jedoch die Beobachtung, dass die Polyaminosäure bei Verwendung der 2-phasigen-Bedingungen in einem separaten Verfahren voraktiviert werden muss, um schnelle Reaktionszeiten und hohe Enantiomerenüberschüsse zu erreichen. Für diese Voraktivierung, die durch Rühren in einer Toluol/NaOH-Lösung erfolgt, werden mehrere Tage benötigt. Gemäß *Tetrahedron Lett.*, **1998,** 39, 9297-9300 erhält man dann den benötigten voraktivierten Katalysator nach einer Wasch- und Trockenprozedur. Die so voraktivierte Polyaminosäure bildet jedoch unter den 2-phasigen-Bedingungen eine Paste, welche die Durchmischung während der Reaktion sowie die nachfolgende Aufarbeitung erschwert. Gemäß EP-A-1 006 127 kann dieses Problem durch eine Adsorption der aktivierten Polyaminosäure an einen festen Träger gelöst werden. Auf Silicagel geträgerte Polyaminosäuren werden als SCAT (silica adsorbed catalysts) bezeichnet.

Ein weiterer Nachteil der 2-phasigen-Bedingungen besteht allerding darin, dass der Einsatz von teuren, nicht-nucleophilen Basen (z.B. DBU) notwendig ist, um die Reaktion zu ermöglichen.

Gemäß EP-A-1 006 111 besteht eine weitere Variante der Juliá-Colonna-Epoxidierung darin, dass die aktivierte Polyaminosäure in Gegenwart von Wasser, einem wassermischbaren Lösungsmittel (z.B. 1,2-Dimethoxyethan) und Natriumpercarbonat die enantioselektive Epoxidierung katalysiert. Aufgrund der Verwendung wassermischbarer Lösungsmittel gestaltet sich bei diesem Verfahren die Aufarbeitung (Extraktion) umständlich.

Bei der Juliá-Colonna-Epoxidierung hängt die Reaktionsgeschwindigkeit und der erreichbare Enantiomerenüberschuss (ee) stark von der verwendeten Polyaminosäure und der Art ihrer Herstellung ab (*Chirality*, **1997,** *9*, 198-202). Um etwa vergleichbare Ergebnisse zu erhalten, wird zur Entwicklung und Beschreibung neuer Methoden in der Literatur durchgängig ein Standardsystem mit Poly-L-leucin (pll) als Katalysator und *trans*-Chalkon als Edukt verwendet. Neben D- oder L-Polyleucin werden jedoch auch andere Polyaminosäuren wie z.B. D- oder L Neopentylglycin mit Erfolg verwendet (EP-A- 1 006 127).

Die Aufgabe der vorliegenden Erfindung bestand darin, ein Verfahren bereitzustellen, welches die Polyaminosäure-katalysierte enantioselektive Epoxidierung von α,β-ungesättigten Enonen und α,β-ungesättigten Sulfonen ermöglicht, aber nicht den Nachteilen der oben beschriebenen Varianten der Juliä-Colonna-Epoxidierung unterliegt. Insbesondere sollte eine schnelle, breit anwendbare Methode gefunden werden, welche eine separat auszuführende Voraktivierung der Polyaminosäure, die Verwendung teurer Basen und Oxidationsmittel sowie potentiell problematische Arten der Reaktionsführung sowie der Aufarbeitung vermeidet. Gleichzeitig sollte das Verfahren Vorteile hinsichtlich der Raum/Zeit-Ausbeute, der Handhabbarkeit, der Ökonomie und Ökologie im technischen Maßstab bringen.

Überraschenderweise wurde nun gefunden, dass man die Epoxidierung von α,β-ungesättigten Enonen und α,β-ungesättigten Sulfonen unter Einsatz spezieller Phasentransferkatalysatoren unter dreiphasigen Bedingungen mit substantiell geringeren Reaktionszeiten bei gleichzeitig noch höheren Enantiomerenüberschüssen durchführen kann.

Gegenstand der Erfindung ist somit ein Verfahren zur Epoxidierung von α,β-ungesättigten Enonen oder α,β-ungesättigten Sulfonen in Gegenwart

(1) einer wasserlöslichen Base,
(2) eines Oxidationsmittels,
(3) einer diastereomeren- und enantiomerenangereicherten Homo-Polyaminosäure als Katalysator,
(4) Wasser und
(5) eines mit Wasser nicht oder nur begrenzt mischbaren organischen Lösungsmittels,
dadurch gekennzeichnet, dass zusätzlich
(6) in Gegenwart eines Phasentransferkatalysators der Formel (I)

$$(R^1R^2R^3R^4A)^+X^- \qquad (I)$$

gearbeitet wird, wobei

| | |
|---|---|
| A | für N oder P steht |
| $X^-$ | für ein anorganisches oder organisches Anion steht, |
| $R^1$ , $R^2$, $R^3$ und $R^4$ | gleich oder verschieden sind und für Alkyl-, Aryl-, Aralkyl-, Cycloalkyl- oder Heteroaryl-Reste stehen, die durch einen oder mehrere, gleiche oder verschiedene Halogen-Reste substituiert sein können, oder aber jeweils zwei Reste unter Einbindung von A einen $C_4$-$C_6$-Cycloalkyl-Ring bilden können, |

wobei

(i) die Summe der in den Resten $R^1$, $R^2$, $R^3$ und $R^4$ enthaltenen Kohlenstoff- und Heteroatome mindestens 13 beträgt und
(ii) die Zugänglichkeit q des Phasentransferkatalysators im Bereich von 0,6-1,3 liegt, wobei sich q durch folgende Formel ergibt:

$$q = \sum_{x=1}^{4} 1 / (\text{Summe der Kohlenstoff- und Heteroatome in } R^x)]$$

Als wesentliches Merkmal umfasst das erfindungsgemäße Verfahren den Einsatz von speziellen Phasentransferkatalysatoren:

Die Messgröße der sogenannten Zugänglichkeit q ist ein empirischer Parameter eines gegebenen Phasentransferkatalysators der allgemeinen Formel (I), der in der Literatur bereits für Tetraalkylammoniumsalze beschrieben ist (*ACS Symp. Ser.*, **1997,** *659,* 100-102).

Die im erfindungsgemäßen Verfahren eingesetzten Phasentransferkatalysatoren besitzen eine Zugänglichkeit q im Bereich von 0,6-1,3, bevorzugt im Bereich von 0,7-1,3 und insbesondere im Bereich von 0,8-1,2.

In der allgemeinen Formel (I) steht $X^-$ bevorzugt für $F^-$, $Cl^-$, $Br^-$, $I^-$, $OH^-$, $NO_3^-$, $HSO_4^-$, $SO_4^-$, $CH_3COO^-$, $CF_3COO^-$, $C_2H_5COO^-$, $C_3H_7COO^-$ , $CF_3SO_3^-$ oder $C_4F_9SO_3^-$.

Bewährt haben sich solche Phasentransferkatalysatoren der allgemeinen Formel (I), bei denen A und $X^-$ die oben genannten Bedeutungen besitzen, $R^1$, $R^2$, $R^3$ und $R^4$ gleich oder verschieden sind und für $C_1$-$C_{18}$-Alkyl, $C_6$-$C_{18}$-Aryl, $C_7$-$C_{19}$-Aralkyl, $C_5$-$C_7$-Cycloalkyl oder $C_3$-$C_{18}$-Heteroaryl stehen und gleichzeitig die oben genannten Bedingungen (i) und (ii) erfüllt sind.

Besonders geeignet sind $((C_4H_9)_4N)^+Hal^-$, insbesondere $((C_4H_9)_4N)^+Br^-$, $((C_4H_9)_4P)^+Hal^-$, insbesondere $((C_4H_9)_4P)^+Br^-$, oder $((C_4H_9)_4N)^+HSO_4^-$ als Phasentransferkatalysatoren.

Phasentransferkatalysatoren wie Aliquat® 336 ($[(CH_3)(C_8H_{17})_3N^+]Cl^-$) und Aliquat® 175 ($[(CH_3)(C_4H_9)_3N^+]Cl^-$), deren Zugänglichkeit ausserhalb des Wertebereiches von 0,6-1,3 liegt, bzw. Phasentransferkatalysatoren wie PEG 400 führen dagegen nicht zu den Vorteilen des erfindungsgemäßen Verfahrens. Bei ihrer Verwendung werden die Zielprodukte nur mit schlechtem Enantiomerenüberschuss bzw. schlechter Raum/Zeit-Ausbeute erhalten.

Die erfindungsgemäß einzusetzenden Phasentransferkatalysatoren sind üblicherweise käuflich erhältlich oder aber nach dem Fachmann geläufigen Methoden herstellbar.

Die Menge des zugesetzten Phasentransferkatalysators ist nicht kritisch und liegt üblicherweise im Bereich von 0.1 - 20 mol%, bevorzugt im Bereich von 0,5-15 mol%, besonders bevorzugt im Bereich von 0,5-11 mol%, jeweils bezogen auf das eingesetzte $\alpha,\beta$-ungesättigte Enon oder $\alpha,\beta$-ungesättigte Sulfon. Bei Mengen, die noch geringer sind als 0,1 mol%, ist jedoch zu beobachten, dass bei unverändert hohem Enantiomerenüberschuss die Reaktionsgeschwindigkeit deutlich abnimmt.

Als $\alpha,\beta$-ungesättigte Enone oder $\alpha,\beta$-ungesättigte Sulfone können Verbindungen der allgemeinen Formel (II) eingesetzt werden

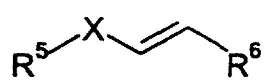

**(II)**

worin

X für (C=O) oder ($SO_2$) steht und

$R^5$ und $R^6$ gleich oder verschieden sind und ($C_1$-$C_{18}$)-Alkyl, ($C_2$-$C_{18}$-Alkenyl, ($C_2$-$C_{18}$)-Alkinyl, ($C_3$-$C_8$)Cycloalkyl, ($C_6$-$C_{18}$)-Aryl, ($C_7$-$C_{19}$)-Aralkyl, ($C_1$-$C_{18}$)-Heteroaryl oder ($C_2$-$C_{19}$)-Heteroaralkyl bedeuten,
wobei die für $R^5$ und $R^6$ genannten Reste einfach oder mehrfach mit gleichen oder verschiedenen Resten $R^7$, Halogen, $NO_2$, $NR^7R^8$, $PO_{0\text{-}3}R^7R^8$, $SO_{0\text{-}3}R^7$, $OR^7$, $CO_2R^7$, $CONHR^7$ oder $COR^7$ substituiert sein können und gegebenenfalls eine oder mehrere $CH_2$-Gruppen in den Resten $R^5$ und $R^6$ durch O, $SO_{0\text{-}2}$, $NR^7$ oder $PO_{0\text{-}2}R^7$ substituiert sind,
wobei $R^7$ und $R^8$ gleich oder verschieden sind und H, ($C_1$-$C_{18}$)-Alkyl, ($C_2$-$C_{18}$)-Alkenyl, ($C_2$-$C_{18}$)

-Alkinyl, $(C_3-C_8)$-Cycloalkyl, $(C_6-C_{18})$-Aryl, $(C_1-C_{18})$-Heteroaryl, $(C_1-C_8)$-Alkyl-$(C_6-C_8)$-Aryl, $(C_1-C_8)$-Alkyl-$(C_1-C_{19})$-Heteroaryl, $(C_1-C_8)$-Alkyl-$(C_3-C_8)$-Cycloakyl bedeuten und diese Reste $R^7$ und $R^8$ einfach oder mehrfach mit gleichen oder verschiedenen Halogenresten substituiert sein können.

Unter einem $(C_1-C_{18})$-Alkylrest wird im Rahmen der Erfindung ein Rest mit 1 bis 18 gesättigten C-Atomen verstanden, der beliebige Verzweigungen aufweisen kann. Insbesondere sind unter dieser Gruppe die Reste Methyl, Ethyl, *n*-Propyl, Isopropyl, *n*-Butyl, Isobutyl, *sec*-Butyl, *tert*-Butyl, Pentyl und Hexyl subsumierbar.

Ein $(C_2-C_{18})$-Alkenylrest weist die für den $(C_1-C_{18})$-Alkylrest genannten Merkmale auf, wobei innerhalb des Restes mindestens eine Doppelbindung vorhanden sein muss.

Ein $(C_2-C_{18})$-Alkinylrest weist die für den $(C_1-C_{18})$-Alkylrest genannten Merkmale auf, wobei innerhalb des Restes mindestens eine Dreifachbindung vorhanden sein muss.

Unter einem $(C_3-C_8)$-Cycloalkylrest wird ein cylischer Alkylrest mit 3 bis 8 C-Atomen und gegebenenfalls beliebiger Verzweigung verstanden. Insbesondere zählen hierzu Reste wie Cyclopropyl, Cyclobutyl, Cyclopentyl, Cyclohexyl und Cycloheptyl. In diesem Rest kann eine oder mehrere Doppelbindungen vorhanden sein.

Unter einem $(C_6-C_{18})$-Arylrest wird ein aromatischer Rest mit 6 bis 18 C-Atomen verstanden. Insbesondere zählen hierzu Reste wie Phenyl-, Naphthyl-, Anthryl- und Phenanthryl.

Unter einem $(C_7-C_{19})$-Aralkylrest wird ein über einen $(C_1-C_8)$-Alkylrest an das Molekül gebundener $(C_6-C_{18})$-Arylrest verstanden.

Ein $(C_1-C_{18})$-Heteroarylrest bezeichnet im Rahmen der Erfindung ein fünf-, sechs- oder siebengliedriges aromatisches Ringsystem mit 1 bis 18 C-Atomen, welches ein oder mehrere Heteroatome, bevorzugt N, O oder S im Ring aufweist. Zu diesen Heteroarylresten zählen z.B. 1-, 2-, 3-Furyl, 1-, 2-, 3-Pyrrol, 1-, 2-, 3-Thienyl, 2-, 3-, 4-Pyridyl, 2-, 3-, 4-, 5-, 6-, 7-Indolyl, 3-, 4-, 5-Pyrazolyl, 2-, 4-, 5-Imidazolyl, 1-, 3-, 4-, 5-Triazolyl, 1-, 4-, 5-Tetrazolyl, Acridinyl, Chinolinyl, Phenanthridinyl, 2-, 4-, 5-, 6-Pyrimidinyl und 4-, 5-, 6-, 7-(1-Aza)-indolizinyl.

Unter einem $(C_2-C_{19})$-Heteroaralkylrest wird ein dem $(C_7-C_{19})$-Aralkylrest entsprechendes heteroaromatisches System verstanden.

Unter Halogen oder auch Hal versteht man im Kontext dieser Erfindung Fluor, Chlor, Brom und Iod.

Als Substrate des erfindungsgemäßen Verfahrens werden bevorzugt $\alpha,\beta$-ungesättigte Enone oder $\alpha,\beta$-ungesättigte Sulfone der allgemeinen Formel (II) eingesetzt, in denen

$R^5$ und $R^6$ gleich oder verschieden sind und $(C_1-C_{12})$-Alkyl, $(C_2-C_{12})$-Alkenyl, $(C_2-C_{12})$-Alkinyl, $(C_5-C_8)$-Cycloalkyl, $(C_6-C_{12})$-Aryl oder $(C_1-C_{12})$-Heteroaryl bedeuten, wobei die zuvor genannten Reste einfach oder mehrfach mit gleichen oder verschiedenen Resten $R^7$, Halogen, $NO_2$, $NR^7R^8$, $PO_{0-3}R^7R^8$ oder $OR^7$ substituiert sein können und $R^7$ und $R^8$ die für die allgemeine Formel (II) zuvor angegebene Bedeutungen besitzen.

Besonders bevorzugt werden als Substrate des erfindungsgemäßen Verfahrens $\alpha,\beta$-ungesättigte Enone oder $\alpha,\beta$-ungesättigte Sulfone der allgemeinen Formel (II) eingesetzt, in denen

$R^5$ und $R^6$ gleich oder verschieden sind und $(C_1-C_{12})$-Alkyl, $(C_2-C_{12})$-Alkenyl, $(C_2-C_{12})$-Alkinyl, $(C_5-C_8)$-Cycloalkyl, $(C_6-C_{12})$-Aryl oder $(C_1-C_{12})$-Heteroaryl bedeuten, wobei die zuvor genannten Reste einfach oder mehrfach mit gleichen oder verschiedenen Resten $R^7$, Halogen, $NO_2$, $NR^7R^8$, $PO_{0-3}R^7R^8$ oder $OR^7$ substituiert sein können und $R^7$ und $R^8$ die für die allgemeine Formel (II) zuvor angegebene Bedeutungen besitzen,

mit der Maßgabe, dass mindestens einer der Reste $R^5$ oder $R^6$ einen $(C_2-C_{12})$-Alkenyl-, $(C_2-C_{12})$-Alkinyl-, $(C_6-C_{12})$-Aryl- oder $(C_1-C_{12})$-Heteroarylrest darstellt.

Insbesondere ist es bevorzugt, Substrate der allgemeinen Formel (III) der erfindungsgemäßen Epoxidierung zu unterwerfen:

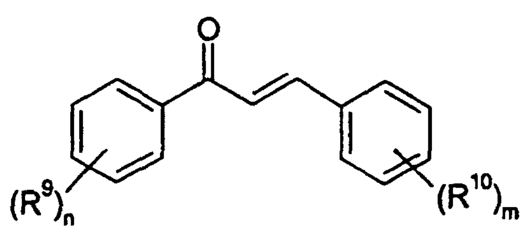

(III)

wobei

n und m gleich oder verschieden sind und für die Zahlen 0, 1, 2 oder 3 stehen,

und

$R^9$ und $R^{10}$ gleich oder verschieden sind und $NR^7R^8$, $NO_2$, $OR^7$, ($C_1$-$C_{12}$)-Alkyl, ($C_2$-$C_{12}$)-Alkenyl, ($C_2$-$C_{12}$)-Alkinyl, ($C_5$-$C_8$)-Cycloalkyl, ($C_6$-$C_{12}$)-Aryl oder ($C_1$-$C_{12}$)-Heteroaryl bedeuten, wobei diese Reste $R^9$ und $R^{10}$ einfach oder mehrfach mit gleichen oder verschiedenen Halogenresten substituiert sein können und $R^7$ und $R^8$ die zuvor für Formel (II) genannten Bedeutungen besitzen.

Das erfindungsgemäße Verfahren zur Herstellung der enantiomerenangereicherten Epoxide wird in Gegenwart von diastereomeren- und enantiomerenangereicherten Homo-Polyaminosäuren als Katalysator durchgeführt. Hierbei können die verschiedensten diastereomeren- und enantiomerenangereicherten Homo-Polyaminosäuren zum Einsatz kommen. Vorzugsweise werden Homo-Polyaminosäuren aus der Gruppe Polyneopentylglycin, Polyleucin, Polyisoleucin, Polyvalin und Polyalanin sowie Polyphenylalanin verwendet. Aus dieser Gruppe sind Polyneopentylglycin und Polyleucin am meisten zu bevorzugen.

Die Kettenlänge der Polyaminosäuren ist dabei üblicherweise so zu wählen, dass einerseits die chirale Induktion bei der Reaktion nicht beeinträchtigt wird und andererseits die Kosten zur Synthese der Polyaminosäuren nicht zu groß werden. Vorzugsweise liegt die Kettenlänge der Homo-Polyaminosäuren im Bereich von 5 bis 100 Aminosäurewiederholungseinheiten, vorzugsweise im Bereich von 7 bis 50 Aminosäurenwiederholungseinheiten. Ganz besonders bevorzugt sind Homo-Polyaminosäuren mit 10 bis 40 Aminosäuren.

Die einzusetzenden Homo-Polyaminosäuren werden vor der Epoxidierung keiner separaten Voraktivierung mit Zwischenisolierung unterzogen und auch nicht auf einen anorganischen Träger aufgebracht. Dies erhöht die wirtschaftliche Attraktivität des Verfahrens erheblich und erleichert auch die technische Durchführung.

Die Homo-Polyaminosäuren können entweder als solche unverändert in die Reaktion eingesetzt werden oder aber zuvor mit polyfunktionellen Aminen vernetzt oder durch andere organische Polymere kettenverlängert werden. Für eine Vernetzung setzt man vorteilhafterweise als vernetzende Amine Diaminoalkane, bevorzugt 1,3-Diaminopropan, oder quervernetztes Hydroxy- oder Aminopolystyrol (CLAMPS, kommerziell erhältlich) ein. Als Polymervergrößerer kommen bevorzugt auf Polyethylenglykol oder Polystyrol basierende Nucleophile in Frage: Derart veränderte Polyaminosäuren sind in *Chem. Commun*., **1998,** 1159-1160 und *Tetrahedron: Asymmetry*, **1997,** *8*, 3163-3173 dargestellt.

Die bei der Epoxidierung einzusetzenden Homo-Polyaminosäuren selber können nach Methoden des Standes der Technik hergestellt werden (*J. Org. Chem*., **1993,** *58,* 6247-6254 oder *Chirality*, **1997,** *9*, 198-202). Die Methode ist auf beide optischen Antipoden der Aminosäuren anzuwenden. Der Einsatz einer bestimmten Antipode einer Polyaminosäure korreliert mit der Stereochemie des Epoxids, d.h. eine Poly-L-aminosäure führt zur optischen Antipode des Epoxids, das mit einer Poly-D-aminosäure erhalten wird.

Die Menge der eingesetzten Homo-Polyaminosäure ist nicht kritisch und liegt üblicherweise im Bereich von 0.0001 - 40 mol%, bevorzugt im Bereich von 0,001-20 mol%, besonders bevorzugt im Bereich von 0,01-15 mol%, und insbesondere im Bereich von 1 bis 15 mol-%, jeweils bezogen auf das eingesetzte $\alpha,\beta$-ungesättigte Enon oder $\alpha,\beta$-ungesättigte Sulfon.

Als Oxidationsmittel dienen in der Regel Peroxide, Persäuren oder anorganische Oxidationsmittel wie Natriumhypochlorit oder Natriumpercarbonat. Bevorzugt sind Peroxide, Persäuren oder Natriumhypochlorit. Besonders bevorzugt wird eine wässrige $H_2O_2$-Lösung eingesetzt. Diese wässrige Lösung kann dabei alle üblichen Konzentrationen haben. Weitere bei dieser Reaktion einzusetzende Oxidationsmittel sind die in *Methoden Org. Chem. (Houben-Weyl),* Band 4/1a+b, 59-319 sowie die in *Oxidation in Organic Chemistry*, ACS Monograph 186, Washington DC, **1990,** 1-47 genannten Verbindungen.

Die Menge des eingesetzten Oxidationsmittels kann in breiten Grenzen von 1 - 40 Äquivalenten variiert werden. Überraschender- und vorteilhafterweise gelingt die erfindungsgemäße Umsetzung bei weiterhin niedrigen Reaktionszeiten und hohen Enantiomerenüberschüssen auch mit relativ geringen Mengen Oxidationsmittel im Bereich von 1 - 10 Äquivalenten, bevorzugt von 1-3 Äquivalenten, besonders bevorzugt von 1,1 - 2,5 Äquivalenten.

Das erfindungsgemäße Verfahren wird in Gegenwart einer wasserlöslichen Base durchgeführt. Bewährt hat sich hierbei der Einsatz von Alkalimetallhydroxiden, wie NaOH, KOH oder LiOH. Die Base wird üblicherweise in Form einer wässrigen Lösung eingesetzt.

Die Menge der eingesetzten Base kann in breiten Grenzen von 0,1-10 Äquivalenten variiert werden. Überraschender- und vorteilhafterweise gelingt die erfindungsgemäße Umsetzung bei weiterhin niedrigen Reaktionszeiten und hohen Enantiomerenüberschüssen auch mit relativ geringen Mengen Base im Bereich von 0,5-5 Äquivalenten, bevorzugt 0,8-2 Äquivalenten.

Das erfindungsgemäße Verfahren wird unter Einsatz eines nicht oder nur begrenzt mit Wasser mischbaren Lösungsmittels durchgeführt. Als begrenzt mit Wasser mischbar wird ein Lösungsmittel im Kontext dieser Erfindung erachtet, wenn eine Mischung aus dem organischen Lösungsmittel und Wasser bei 20°C nicht mehr als 20 Gew.%, bevorzugt nicht mehr als 10 Gew.% und insbesondere nicht mehr als 8 Gew.% Wasser enthalten kann, um einphasig zu bleiben.

Als organische Lösungsmittel kommen allgemein unsubstituierte oder substituierte aromatische Kohlenwasserstoffe, aliphatische Kohlenwasserstoffe, Halogenalkane sowie Ether in Betracht. Geeignet sind besonders Toluol, Xylol, Hexan, *tert.*-Butylmethylether, Diethylether, Chloroform und Methylenchlorid.

Bei der Optimierung des Enantiomerenüberschusses und der Reaktionsgeschwindigkeit in Abhängigkeit vom verwendeten Lösungsmittel werden ähnliche Effekte beobachtet wie unter den klassischen 3-phasigen-Bedingungen, d.h. hohe Enantiomerenüberschüsse werden insbesondere in aromatischen Kohlenwasserstoffen wie Toluol erhalten, während besonders geringe Reaktionszeiten in Ethern, wie *tert.*-Butylmethylether, oder Halogenalkanen, wie Chloroform, erreicht werden.

Gefunden wird ferner, dass die Homo-Polyaminosäure pll in *tert*.-Butylmethylether aggregiert. Für eine kontinuierliche Reaktionsführung ist daher der *tert*.-Butylmethylether ein interessantes und geeignetes Lösungsmittel.

Die Temperatur, die bei der Epoxidierung verwendet wird, liegt im allgemeinen im Bereich von -10 bis +50 °C, bevorzugt im Bereich von 0 bis +40 °C und insbesondere bei +10 bis +30 °C.

Der pH-Wert, welcher während der Reaktion eingestellt wird, kann so gewählt werden, dass ein Überschuss an deprotoniertem $H_2O_2$ verglichen mit nicht deprotoniertem $H_2O_2$ vorhanden ist. Andererseits sollte der pH-Wert bei der Reaktion auch nicht so hoch gewählt werden, dass die eingesetzten organischen Verbindungen Schaden nehmen. Vorzugsweise liegt der pH-Wert im Bereich von 7 - 14, bevorzugt im Bereich von 7,5 - 13.

Der Wasseranteil des Systems resultiert üblicherweise daraus, dass wie bereits beschrieben einzelne Reaktionskomponenten des Systems, wie die Base und das Oxidationsmittel, in Form wässriger Lösungen eingesetzt werden. Insgesamt liegt der Wassergehalt des Reaktionsgemisches im Bereich von 1-70 Gew.%, bevorzugt im Bereich von 5-50 Gew.%, bezogen auf das gesamte Reaktionsgemisch.

Im Hinblick auf die Durchführung der Reaktion wird üblicherweise so verfahren, dass die Base, die Homo-Polyaminosäure, der Phasentransferkatalysator, das Lösungsmittel, Wasser und das Substrat zusammengegeben werden und anschließend das Oxidationsmittel zugesetzt wird.

Das erfindungsgemäße Verfahren zeichnet sich durch stark verringerte Reaktionszeiten aus. Anstelle von Tagen reichen bereits wenige Stunden oder sogar nur Minuten aus, um die Epoxidierung der $\alpha,\beta$-ungesättigten Enone und der $\alpha,\beta$-ungesättigten Sulfone mit hohem Umsatz und hoher Enantioselektivität zu erreichen.

Die erfindungsgemäße Verwendung des Phasentransferkatalysators als Co-Katalysator erlaubt es, die notwendigen Mengen des Oxidationsmittels und der Base sehr deutlich zu reduzieren, ohne dass hierdurch die Reaktionsgeschwindigkeit, Umsatz oder Enantiomerenüberschuss negativ beeinflußt werden. Von Vorteil ist zudem, dass besonders kostengünstige Basen und Oxidationsmittel eingesetzt werden können.

Aufgrund der sehr kurzen Reaktionszeiten sind über das erfindungsgemäße Verfahren erstmals auch Hydroxid-empfindliche Substrate, die nach den klassischen 3-phasigen-Bedingungen (*J. Chem. Soc., Perkin Trans. 1*, **1997,** 3501-3507) nicht erfolgreich epoxidiert werden können, einer enantioselektiven Epoxidierung unter wässrigen, dreiphasigen Bedingungen zugänglich.

## <u>Beispiele</u>

Der Herstellungsprozess für Polyaminosäuren liefert oft Katalysatoren für die Juliä-Colonna Epoxidierung, die eine stark unterschiedliche katalytische Aktivität aufweisen (*Chirality*, **1997,** *9*, 198-202). Der Umsatz pro Zeiteinheit und der Enantiomerenüberschuss lassen sich für ein bestimmtes Substrat nur vergleichen, wenn für die Epoxidierungsreaktion dieselbe Polyaminosäure-Charge verwendet wird. Aus diesem Grund ist ein direkter Vergleich von neuen Ergebnissen mit in der Literatur publizierten Resultaten nicht möglich, da eben zwangsläufig unterschiedliche Katalysatorchargen verwendet werden. Aus diesem Grund wurden bei den nachfolgenden Beispielgruppen I-VIII jeweils einheitliche Polyleucin-Chargen verwendet (sowohl bei den erfindungsgemäßen Beispielen als auch bei den entsprechenden Vergleichsbeispiele).

In allen nachfolgenden Beispielen werden der Umsatz und der Enantiomerenüberschuss (*ee*-Wert) gemäß literaturbekannten Verfahren mittels HPLC an einer chiral, nicht-racemischen Phase bestimmt (UV-Detektion).

**Beispielgruppe I:**

**Beispiele 1-3 und Vergleichsbeispiele VB 4-9**

**Epoxidierung von *trans*-Chalkon mit verschiedenen Phasentransferkatalysatoren**

In den nachfolgenden Beispielen wird der Einfluss unterschiedlicher Phasentransferkatalysatoren (PTC) mit unterschiedlichen Zugänglichkeiten auf die Epoxidierung von *trans*-Chalkon (1) zum Epoxychalkon (2) unter dreiphasigen Reaktionsbedinzungen gezeigt.

**Schema 1:**

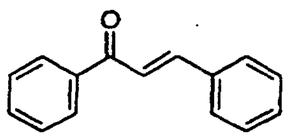

(1)

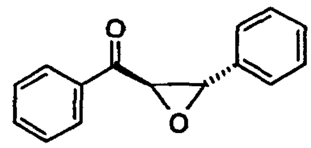

(2)

100 mg nicht voraktivierter Polyaminosäure pll (11 mol%), 0.24 mmol *trans*-Chalkon sowie 8.5 mg $(Bu_4N)^+Br^-$ (oder 11 mol% eines anderen PTC) werden in einer Mischung aus 0.6 ml Toluol und 0,2 ml NaOH (eingesetzt als 5 molare Lösung, entspricht 4,2 Äquivalenten) suspendiert. Anschließend werden 0,7 ml $H_2O_2$ (eingesetzt als 30%ige wässrige Lösung, entspricht 28,5 Äquivalenten) zugesetzt. Diese Mischung wird anschließend bei Raumtemperatur unter Rühren umgesetzt. Nach beendeter Reaktion (bzw. einer gewählten Reaktionszeit) wird die Reaktionsmischung mit 2 ml Ethylacetat verdünnt und anschließend zentrifugiert. Der Überstand wird dann langsam in eine gerührte, eiskalte wässrige $NaHSO_3$-Lösung eingetragen (4 ml, 20%ige Lösung). Nach der Phasentrennung wird die organische Phase über Natriumsulfat getrocknet und unter reduziertem Druck eingeengt. Die Ergebnisse dieser Epoxidierung sind in der nachfolgenden Tabelle 1 zusammengestellt.

Tabelle 1

| **Einfluß von PTCs als Co-Katalysatoren** | | | | | | |
|---|---|---|---|---|---|---|
| Beispiel | PTC | Reaktionszeit [h] | C# | q | Umsatz [%] | ee [%] |
| 1 | $(Bu_4N)^+Br^-$ | 1.5 | 16 | 1.00 | >99 | 94 |
| 2 | $(Bu_4P)^+Br^-$ | 1.0 | 16 | 1.00 | 91 | 87 |
| 3 | $(Bu_4N)^+HSO_4^-$ | 1.0 | 16 | 1.00 | 35 | 90 |
| VB 4 | --- | 1.5 | --- | --- | 2 | nicht bestimmt |
| VB 5 | Aliquat® 175 = $(MeBu_3N)^+Cl^-$ | 1.0 | 13 | 1.75 | 9 | 77 |
| VB 6 | PEG400 | 1.0 | | | 1 | nicht bestimmt |
| VB 7 | $(Et_3BnN)^+Cl^-$ | 1.0 | 13 | 1,64 | 0 | nicht bestimmt |
| VB 8 | Aliquat® 336 = $(MeOct_3N)^+Cl^-$ | 1.0 | 25 | 1.38 | 71 | 14 |
| VB 9 | $(Oct_4N)^+Br^-$ | 0,5 | 32 | 0.50 | 75 | 7 |

q Zugänglichkeit des Phasentransferkatalysators
C# Summe der C- bzw. Heteroatome im eingesetzten Phasentransferkatalysator
Bn Benzyl
Me Methyl
Et Ethyl
Bu n-Butyl
Oct n-Octyl
PEG Polyethylenglycol

**Beispielgruppe II:**

**Beispiele 10-13**

**Untersuchung des Lösungsmitteleinflusses auf die Epoxidierung in Gegenwart von $(Bu_4N)^+Br^-$ als Phasentransferkatalysator**

100 mg nicht voraktivierte Polyaminosäure pll (11 mol%), 0.24 mmol *trans*-Chalkon sowie 8.5 mg $(Bu_4N)^+Br^-$ (oder 11 mol% eines anderen PTC) werden in 0,8 ml des angegebenen Lösungsmittels und 0,2 ml NaOH (eingesetzt als 5 molare Lösung, entspricht 4,2 Äquivalenten) suspendiert. Anschließend werden 0,7 ml $H_2O_2$ (eingesetzt als 30%ige wässrige Lösung, entspricht 28,5 Äquivalenten) zugesetzt. Diese Mischung wird anschließend unter Rühren bei Raumtemperatur umgesetzt. Nach einer Reaktionszeit von 1 Stunde wird die Reaktionsmischung mit 2 ml Ethylacetat verdünnt und anschließend zentrifugiert. Der Überstand wird dann langsam in eine gerührte, eiskalte wässrige $NaHSO_3$-Lösung eingetragen (4 ml, 20%ige Lösung). Nach der Phasentrennung wird die organische Phase über Natriumsulfat getrocknet und unter reduziertem Druck eingeengt. Die Ergebnisse dieser Beispiele sind in der nachfolgenden Tabelle 2 zusammengefasst.

Tabelle 2

| **Einfluss des Lösungsmittels auf die Epoxidierung von *trans*-Chalkon in Gegenwart von $(Bu_4N)^+Br^-$ als PTC** | | | |
|---|---|---|---|
| Beispiel | Lösungsmittel | Umsatz [%] | ee [%] |
| 10 | Toluol | 19 | 90 |
| 11 | *tert*-Butylmethylether | 88 | 77 |
| 12 | *n*-Hexan | 19 | 68 |
| 13 | $CHCl_3$ | 70 | 60 |

**Beispielgruppe III:**

**Beispiele 14-17**

**Epoxidierung von *trans*-Chalkon in Gegenwart unterschiedlicher Mengen von $(Bu_4N)^+Br^-$ als Phasentransferkatalysator**

0,27 g nicht voraktivierte Polyaminosäure pll (0,3 mol%), 5.0 g *trans*-Chalkon sowie $(Bu_4N)^+Br^-$ in unterschiedlichen Mengen (s. Tabelle 3) werden in 20 ml Toluol und 7.2 ml NaOH (eingesetzt als 5 molare Lösung, entspricht 1,5 Äquivalenten) suspendiert. Anschließend werden 3.7 ml $H_2O_2$ (eingesetzt als 30%ige wässrige Lösung, entspricht 1.5 Äquivalenten) zugesetzt. Diese Mischung wird anschließend unter Rühren bei Raumtemperatur umgesetzt. Nach einer Reaktionszeit von 2 Stunden wird die Reaktionsmischung mit 50 ml Ethylacetat verdünnt und anschließend zentrifugiert. Der Überstand wird dann langsam in eine gerührte, eiskalte wässrige $NaHSO_3$-Lösung eingetragen (100 ml, 20%ige Lösung). Nach der Phasentrennung wird die organische Phase über Natriumsulfat getrocknet und unter reduziertem Druck eingeengt. Die Ergebnisse dieser Beispiele sind in der nachfolgenden Tabelle 3 zusammengefasst.

Tabelle 3

| **Einfluss unterschiedlicher Phasentransferkatalysator-Mengen auf die Epoxidierung von *trans*-Chalkon** | | | | |
|---|---|---|---|---|
| Beispiel | $(Bu_4N)^+Br^-$ [g] | $(Bu_4N)^+Br^-$ [mol%] | Umsatz [%] | ee [%] |
| 14 | 0,23 | 3 | 95 | 93 |
| 15 | 0,15 | 2 | 70 | 93 |
| 16 | 0,06 | 0,8 | 49 | 92 |
| 17 | 0,015 | 0,2 | 19 | 94 |

**Beispielgruppe IV:**

**Beispiel 18 und Vergleichsbeispiel VB 19**

**Epoxidierung von *trans*-Chalkon (1) zu Epoxychalkon (2) gemäß Schema 1 mit NaOCl als Oxidationsmittel**

*Trans*-Chalkon wird in Gegenwart von unaktivierter Polyaminosäure pll (11 mol%), 11 mol.% $(Bu_4N)^+Br^-$ (nur in Beispiel 14), 6 ml NaOCl (eingesetzt als 7.5%ige wässrige Lösung) und Toluol als Lösungsmittel für 1,5 Stunden bei Raumtemperatur zum Epoxychalkon umgesetzt. Die Aufarbeitung erfolgte durch Verdünnen mit 2 ml Ethylacetat, Zentrifugieren sowie anschließendem Trocknent über Natriumsulfat und Einengen des Überstandes. Die erhaltenen Ergebnisse sind in Tabelle 4 enthalten.

Tabelle 4

| **Epoxidierung von *trans*-Chalkon mit wässriger NaOCl-Lösung** | | | |
|---|---|---|---|
| Beispiel | PTC | Umsatz [%] | ee [%] |
| 18 | $(Bu_4N)^+Br^-$ | 32 | 90 |
| VB 19 | --- | 1 | nicht bestimmt |

**Beispiel 20: Epoxidierung von (*E*)-1,2-Dibenzoylethylen (3) zu (4) (dreiphasige Bedingungen mit PTC**

**Schema 2:**

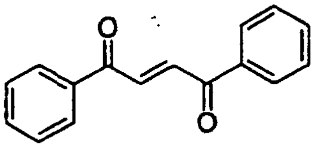

(3)

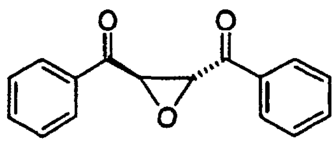

(4)

100 mg nicht voraktivierte pll (11 mol%), 57 mg (*E*)-1,2-Dibenzoylethylen sowie 8.5 mg $(Bu_4N)^+Br^-$ (11 mol%) wurden in einer Mischung aus 0.8 ml Toluol und 63 µl NaOH (eingesetzt als 5 molare wässrige Lösung, entspricht 1.3 Äquivalenten) suspendiert. Anschließend wurden 32 µl $H_2O_2$ (eingesetzt als 30%ige wässrige Lösung, entspricht 1.3 Äquivalenten) zugesetzt. Diese Mischung wurde beim Raumtemperatur unter Rühren umgesetzt. Nach 5 min Reaktionszeit wurde die Reaktionsmischung mit 2 ml Ethylacetat verdünnt und anschließend langsam in eine gerührte, eiskalte wäßrige $NaHSO_3$-Lösung eingetragen (4 ml, 20%ig). Nach Abfiltrieren des Polymers wurde die organische Phase des Filtrats über Natriumsulfat getrocknet und unter verringertem Druck eingeengt. Es wird ein Umsatz von 100% und ein Enantiomerenüberschuß von 72% *ee* erhalten (bestimmt durch Shift-$^1$H-NMR-Experiment mit $Eu(tfc)_3$ als Shift-Reagenz).

**Beispiel 21: Epoxidierung von (*E*)-1-Phenyl-3-(2-pyridinyl)-2-propen-1-on (5) zu (6) (dreiphasige Bedingungen mit PTC)**

**Schema 3:**

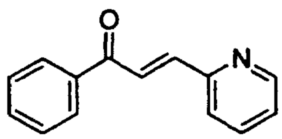

(5)

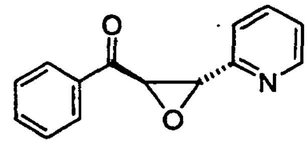

(6)

9 mg nicht voraktivierte pll (0.5 mol%), 100 mg (*E*)-1-Phenyl-3-(2-pyridinyl)-2-propen-1-on sowie 8.5 mg $(Bu_4N)^+Br^-$ (0.3 mol%) wurden in einer Mischung aus 0.2 ml Toluol und 0.14 ml NaOH (eingesetzt als 5 molare wässrige Lösung, entspricht 1.5 Äquivalenten) suspendiert. Anschließend wurden 74 µl $H_2O_2$ (eingesetzt als 30%ige wässrige Lösung, entspricht 1.5 Äquivalenten) zugesetzt. Diese Mischung wurde beim Raumtemperatur unter Rühren umgesetzt. Nach 30 min Reaktionszeit wurde die Reaktionsmischung mit 2 ml Ethylacetat verdünnt und dann langsam in eine gerührte, eiskalte wäßrige $NaHSO_3$-Lösung eingetragen (4 ml, 20%ig). anschließend abfiltriert. Nach Zentrifugation wurde der Überstand abfiltriert und unter verringertem Druck eingeengt. Es wird ein Umsatz von >99% und eine Enantiomerenüberschuß von 84% *ee* erhalten (bestimmt mittels chiraler GC).

In allen nachfolgenden Beispielgruppen wird wiederum das erfindungsgemäße Verfahren mit literaturbekannten Bedingungen, bei denen kein Phasentransferkatalysator zugesetzt wird, verglichen. Hierfür wurden die besten publizierten Bedingungen für die ausgewählten Beispielreaktionen mit der gleichen Polyaminosäure-Charge nachgestellt, die auch für die Durchführung des erfindungsgemäßen Verfahrens eingesetzt wird.

**Beispielgruppe V:**

**Beispiele 22 und Vergleichsbeispiele 23-25**

**Epoxidierung von *trans*-Chalkon (1) zu Epoxychalkon (2) gemäß Schema 1 unter dreiphasigen Bedingungen**

**Beispiel 22 3-phasige Bedingungen mit PTC**

100 mg nicht voraktivierte pll, 50 mg *trans*-Chalkon sowie 8.5 mg $(Bu_4N)^+Br^-$ wurden in einer Mischung aus 0.8 ml Toluol und 62 µl NaOH (eingesetzt als 5 molare wässrige Lösung, entspricht 1.3 Äquivalenten) suspendiert. Anschließend wurden 32µl $H_2O_2$ (eingesetzt als 30%ige wässrige Lösung, entspricht 1.3 Äquivalenten) zugesetzt. Diese Mischung wurde bei Raumtemperatur unter Rühren umgesetzt. Nach 10 min Reaktionszeit wurde die Reaktionsmischung mit 2 ml Ethylacetat verdünnt und anschließend langsam in eine gerührte, eiskalte wässrige $NaHSO_3$-Lösung eingetragen (4 ml, 20%ig ). Nach Zentrifugation wurde der Überstand über Natriumsulfat getrocknet und unter verringertem Druck eingeengt.

**Vergleichsbeispiel VB123 3-phasige Bedingungen ohne PTC**

100 mg nicht voraktivierte pll wurden in einer Mischung aus 0.8 ml Toluol, 0.2 ml NaOH (eingesetzt als 5 molare wässrige Lösung, entspricht 4.2 Äquivalenten) und 0.2 ml $H_2O_2$ (eingesetzt als 30%ige wässrige Lösung) suspendiert. Diese Mischung wurde bei Raumtemperatur für 6 h unter Rühren umgesetzt. Anschließend wurden 50 mg *trans*-Chalkon und weitere 0.5 ml $H_2O_2$ (eingesetzt als 30%ige wässrige Lösung, entspricht einer insgesamt zugesetzten $H_2O_2$- Menge von 28.5 Äquivalenten) zugesetzt. Nach 1 h Reaktionszeit wurde die Reaktionsmischung mit 2 ml Ethylacetat verdünnt und anschließend langsam in eine gerührte, eiskalte wässrige $NaHSO_3$-Lösung eingetragen (4 ml, 20%ig). Nach Zentrifugation wurde der Überstand über Natriumsulfat getrocknet und unter verringertem Druck eingeengt.

**Vergleichsbeispiel VB24 2-phasige Bedingungen ohne PTC**

**a) Separate Voraktivierung des pll**

1 g des Polymers wurde in einem Gemisch aus 5 ml Toluol und 10 ml NaOH (eingesetzt als 5 molare wässrige Lösung) suspendiert und für 5 Tage gerührt. Während dieser Zeit bildete sich ein Gel. Zur Aufarbeitung wurde das Polymer durch Dekantieren isoliert, mit 20 ml Ethanol verrührt und anschließend abfiltriert. Der Filterkuchen (Polymer) wurde mit Wasser neutral gewaschen. Anschließend wurde das Polymer noch dreimal mit Aceton gewaschen und abschließend im Vakuum über $P_2O_5$ getrocknet.

**b) Epoxidierung unter 2-phasigen Bedingungen**

50 mg *trans*-Chalkon, 25 mg Harnstoff-Wasserstoffperoxid-Komplex (UHP, 0.26 mmol, 1.1 Äquivalente) und separat voraktiviertes 94.5 mg pll (11 mol%, Voraktivierung siehe Punkt a)) wurden gemischt, mit 2.7 ml wasserfreiem THF suspendiert und mit 40 µl DBU (1.1 Äquivalente) versetzt. Die Reaktionsmischung wurde bei Raumtemperatur unter Rühren umgesetzt. Nach 10 min Reaktionszeit wurde die Reaktionsmischung mit 2 ml Ethylacetat verdünnt und anschließend zentrifugiert. Der Überstand wurde dann langsam in eine gerührte, eiskalte wäßrige $NaHSO_3$-Lösung eingetragen (4 ml, 20%ig). Nach der Phasentrennung wurde die organische Phase über Natriumsulfat getrocknet und unter verringertem Druck eingeengt.

**Vergleichsbeispiel VB25 SCAT-Bedingungen**

**a) Herstellung von SCAT**

1 g separat voraktiviertes pll (die Voraktivierung erfolgte gemäß Punkt a) der Vorschrift zu Vergleichsbeispiel VB18) und 3.4 g Silicagel 60 (230-400 mesh, Merck) wurden gemischt, in 30 ml wasserfreiem THF suspendiert und unter Lichtausschluß für 48 h langsam gerührt. Die Suspension wurde filtriert und der Rückstand zweimal mit je 10 ml wasserfreiem THF gewaschen. Das Material (*SCAT*) wurde im Vakuum über $P_2O_5$ getrocknet.

**b) Epoxidierung unter SCAT-Bedingungen**

50 mg *trans*-Chalkon, 25 mg Harnstoff-Wasserstoffperoxid-Komplex (UHP, 0.26 mmol, 1.1 Äquivalente) und 419 mg SCAT (11 mol%) wurden gemischt, mit 2.7 ml wasserfreiem THF suspendiert und mit 40 µl DBU (1.1 Äquivalente) versetzt. Die Reaktionsmischung wurde bei Raumtemperatur unter Rühren umgesetzt. Nach 10 min Reaktionszeit wurde die Reaktionsmischung filtriert. Das Filtrat wurde mit 2 ml Etylacetat versetzt und anschließend langsam in eine gerührte, eiskalte wässrige $NaHSO_3$-Lösung eingetragen (4 ml, 20%ig). Nach der Phasentrennung wurde die organische Phase über Natriumsulfat getrocknet und unter verringertem Druck eingeengt.

Die Ergebnisse des Beispiels 22 sowie der Vergleichsbeispiele VB 23-25 sind in der nachfolgenden Tabelle 5 zusammengefasst.

Tabelle 5:

| Beispiel | Bedingungen | PTC | Reaktionszeit [min] | Umsatz [%] | ee [%] |
|---|---|---|---|---|---|
| 22 | erfindungsgemäß | $(Bu_4N^+Br^-$ | 10 | 97 | 94 |
| VB 23 | 3-phasig; nicht erfindungsgemäß | ----- | 60 | 89 | 94 |
| VB 24 | 2-phasig; nicht erfindungsgemäß | ------ | 10 | 28 | 93 |
| VB 25 | 2-phasig, SCAT; nicht erfindungsgemäß | ------ | 10 | 55 | 95 |

**Beispielgruppe VI:**

**Beispiel 26 und Vergleichsbeispiele 27-29**

**Epoxidierung von (*E*)-1-(2-Aminophenyl)-3-phenyl-2-propen-1-on (7) zu (8)**

**Schema 4:**

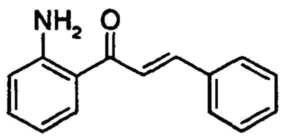

(7)

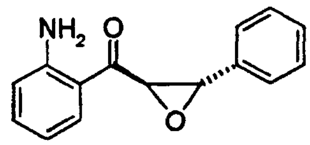

(8)

**Beispiel 26 3-phasige Bedingungen mit PTC**

100 mg nicht voraktivierte pII, 54 mg *trans*-Aminochalkon sowie 8.5 mg $(Bu_4N)^+Br^-$ wurden in einer Mischung aus 0.8 ml Toluol und 200 µl NaOH (eingesetzt als 5 molare wässrige Lösung, entspricht 4.2 Äquivalenten) suspendiert. Anschließend wurden 125 µl $H_2O_2$ (eingesetzt als 30%ige wässrige Lösung, entspricht 5 Äquivalenten) zugesetzt. Diese Mischung wurde bei Raumtemperatur unter Rühren umgesetzt. Nach 10 min Reaktionszeit wurde die Reaktionsmischung mit 2 ml Ethylacetat verdünnt und anschließend zentrifugiert. Der Überstand wurde dann langsam in eine gerührte, eiskalte wässrige $NaHSO_3$-Lösung eingetragen (4 ml, 20%ig). Nach der Phasentrennung wurde die organische Phase über Natriumsulfat getrocknet und unter verringertem Druck eingeengt.

**Vergleichsbeispiel VB 27 3-phasige Bedingungen ohne PTC**

100 mg nicht voraktivierte pII wurden in einer Mischung aus 0.8 ml Toluol, 0.2 ml NaOH (eingesetzt als 5 molare wässrige Lösung, entspricht 4,2 Äquivalenten) und 0.2 ml $H_2O_2$ (eingesetzt als 30%ige wässrige Lösung) suspendiert. Diese Mischung wurde für 6 h unter Rühren umgesetzt. Anschließend wurden 54 mg *trans*-Aminochalkon und weitere 0.5 ml $H_2O_2$ (eingesetzt als 30%ige wässrige Lösung entspricht einer insgesamt zugesetzten $H_2O_2$ -Menge von 28.5 Äquivalenten) zugesetzt. Nach 1 h Reaktionszeit wurde die Reaktionsmischung mit 2 ml Ethylacetat verdünnt und in eine gerührte, eiskalte wäßrige $NaHSO_3$-Lösung eingetragen (4 ml, 20%ig). Anschließend wurde die Mischung zentrifugiert. Der Überstand wurde dann über Natriumsulfat getrocknet und unter verringertem Druck eingeengt.

**Vergleichsbeispiel VB28 2-phasige Bedingungen**

**a) Separate Voraktivierung des pII**

1 g des Polymers wurde in einem Gemisch aus 5 ml Toluol und 10 ml NaOH (eingesetzt als 5 molare wässrige Lösung) suspendiert und für 5 Tage gerührt. Während dieser Zeit bildete sich ein Gel. Zur Aufarbeitung wurde das Polymer durch Dekantieren isoliert, mit 20 ml Ethanol verrührt und anschließend abfiltriert. Der Filterkuchen (Polymer) wurde mit Wasser neutral gewaschen. Anschließend wurde das Polymer noch dreimal mit Aceton gewaschen und abschließend im Vakuum über $P_2O_5$ getrocknet.

**b)Epoxidierung unter 2-phasigen Bedingungen**

54 mg *trans*-Aminochalkon, 25 mg Harnstoff-Wasserstoffperoxid-Komplex (UHP, 0.26 mmol, 1.1 Äquivalente) und separat voraktiviertes 94.5 mg pII (11 mol%, Voraktivierung erfolgte wie oben unter a) beschrieben) wurden gemischt, mit 2.7 ml wasserfreiem THF suspendiert und mit 40 µl DBU (1.1 Äquivalente) versetzt. Die Reaktionsmischung wurde bei Raumtemperatur unter Rühren umgesetzt. Nach 10 min Reaktionszeit wurde die Reaktionsmischung mit 2

ml Ethylacetat verdünnt und anschließend zentrifugiert. Der Überstand wurde langsam in eine gerührte, eiskalte wäßrige $NaHSO_3$-Lösung eingetragen (4 ml, 20%ig). Nach der Phasentrennung wurde die organische Phase über Natriumsulfat getrocknet und unter verringertem Druck eingeengt.

**Vergleichsbeispiel VB29: SCAT-Bedingungen**

**a) Herstellung von SCAT**

1 g separat voraktiviertes pll (Vorschrift siehe Punkt a) des Vergleichsbeispiels VB18) und 3.4 g Silicagel 60 (230-400 mesh, Merck) wurden gemischt, in 30 ml wasserfreiem THF suspendiert und unter Lichtausschluß für 48 h langsam gerührt. Die Suspension wurde filtriert, und der Rückstand zweimal mit je 10 ml wasserfreiem THF gewaschen. Das Material (*SCAT*) wurde im Vakuum über $P_2O_5$ getrocknet.

**b) Epoxidierung unter SCAT-Bedingungen**

54 mg *trans*-Aminochalkon, 25 mg Harnstoff-Wasserstoffperoxid-Komplex (UHP, 0.26 mmol, 1.1 Äquivalent) und 419 mg *SCAT* (11 mol%) wurden gemischt, mit 2.7 ml wasserfreiem THF suspendiert und mit 40 µl DBU (1.1 Äquivalente) versetzt. Die Reaktionsmischung wurde bei Raumtemperatur unter Rühren umgesetzt. Nach 10 min Reaktionszeit wurde die Reaktionsmischung mit 2 ml Ethylacetat verdünnt und anschließend zentrifugiert. Der Überstand wurde langsam in eine gerührte, eiskalte wäßrige $NaHSO_3$-Lösung eingetragen (4 ml, 20%ig). Nach der Phasentrennung wurde über Natriumsulfat die organische Phase getrocknet und unter verringertem Druck eingeengt.

Die Ergebnisse des Beispiels 26 sowie der Vergleichsbeispiele 27-29 sind in der nachfolgenden Tabelle 6 zusammengefasst.

Tabelle 6

| Beispiel | Bedingungen | PTC | Reaktionszeit [min] | Umsatz [%] | ee [%] |
|---|---|---|---|---|---|
| 26 | erfindungsgemäß | $(Bu_4N)^+Br^-$ | 10 | 58 | 87 |
| VB 27 | 3-phasig; nicht erfindungsgemäß | ---- | 60 | 7 | Nicht bestimmt |
| VB 28 | 2-phasig; nicht erfindungsgemäß | ---- | 10 | 14 | Nicht bestimmt |
| VB 29 | 2-phasig/SCAT; nicht erfindungsgemäß | ---- | 10 | 5 | Nicht bestimmt |

**Beispielgruppe VII:**

**Beispiel 30 und Vergleichsbeispiel 31**

**Epoxidierung von (*E*)-1-Cyclopropyl-3-phenyl-2-propen-1-on (9) zu (10)**

**Schema 5:**

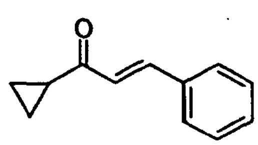

(9)

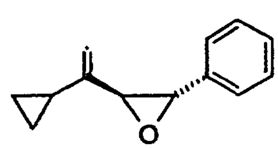

(10)

**Beispiel 30 3-phasige Bedingungen mit PTC**

100 mg nicht voraktivierte pll, 41 mg (*E*)-1-Cyclopropyl-3-phenyl-2-propen-1-on sowie 7.7 mg $(Bu_4N)^+Br^-$ wurden in einer Mischung aus 0.8 ml Toluol und 200 µl NaOH (eingesetzt als 5 molare wässrige Lösung, entspricht 4.2 Äquivalenten) suspendiert. Anschließend wurden 700 µl $H_2O_2$ (eingesetzt als 30%ige wässrige Lösung, entspricht 28.5 Äquivalenten) zugesetzt. Diese Mischung wurde beim Raumtemperatur unter Rühren umgesetzt. Nach 5 h min Reaktionszeit wurde die Reaktionsmischung mit 1 ml Ethylacetat verdünnt und anschließend abfiltriert. Die organische Phase des Filtrats wurde dann langsam in eine gerührte, eiskalte wäßrige $NaHSO_3$-Lösung eingetragen (4 ml, 20%ig). Nach der Phasentrennung wurde die organische Phase über Natriumsulfat getrocknet und unter verringertem Druck eingeengt.

**Vergleichsbeispiel 31 3-phasige Bedingungen ohne PTC**

100 mg nicht voraktivierte pll wurden in einer Mischung aus 0.8 ml Toluol, 0.2 ml NaOH (eingesetzt als 5 molare wässrige Lösung, entspricht 4.2 Äquivalenten) und 0.2 ml $H_2O_2$ (eingesetzt als 30%ige wässrige Lösung) suspendiert. Diese Mischung wurde bei Raumtemperatur für 16 h unter Rühren umgesetzt. Anschließend wurden 41 mg (*E*)-1-Cyclopropyl-3-phenyl-2-propen-1-on und weitere 0.5 ml $H_2O_2$ (eingesetzt als 30%ige wässrige Lösung, entspricht einer insgesamt zugesetzten $H_2O_2$ -Menge von 28.5 Äquivalenten) zugesetzt. Nach 5 h Reaktionszeit wurde die Reaktionsmischung mit 1 ml Ethylacetat verdünnt und anschließend langsam in eine gerührte, eiskalte wässrige $NaHSO_3$-Lösung eingetragen (4 ml, 20%ig). Nach Filtration wurde die organische Phase des Filtrats über Natriumsulfat getrocknet und unter verringertem Druck eingeengt.

Die Ergebnisse des Beispiels 30 und des Vergleichsbeispiels 31 sind in der nachfolgenden Tabelle 7 zusammengefasst.

Tabelle 7

| Beispiel | Bedingungen | PTC | Reaktionszeit [h] | Umsatz [%] | ee [%] |
|---|---|---|---|---|---|
| 30 | erfindungsgemäß | $(Bu_4N)^+Br^-$ | 5 | 40 | 90 |
| VB31 | 3-phasig; nicht erfindungsgemäß | ---- | 5 | 6 | Nicht bestimmt |

**Beispielgruppe VIII:**

**Beispiel 32 und Vergleichsbeispiel 33**

**Epoxidierung von Phenyl-*E*-styrylsulfon (11) zu (12)**

**Schema 6:**

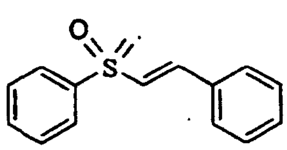

(11)

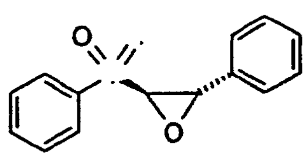

(12)

**Beispiel 32 3-phasige Bedingungen mit PTC**

100 mg nicht voraktivierte pll, 59 mg Phenyl-*E*-styrylsulfon sowie 8.5 mg $(BU_4N)^+Br^-$ wurden in einer Mischung aus 0.8 ml Toluol und 200 µl NaOH (eingesetzt als 5 molare wässrige Lösung, entspricht 4.2 Äquivalenten) suspendiert. Anschließend wurden 125 µl $H_2O_2$ (eingesetzt als 30%ige wässrige Lösung, entspricht 5 Äquivalenten) zugesetzt.

Diese Mischung wurde beim Raumtemperatur unter Rühren umgesetzt. Nach 2 h Reaktionszeit wurde die Reaktionsmischung mit 2 ml Ethylacetat verdünnt und anschließend zentrifugiert. Der Überstand wurde dann langsam in 2 ml Wasser eingetragen. Nach der Phasentrennung wurde die organische Phase über Natriumsulfat getrocknet und unter verringertem Druck eingeengt.

**Vergleichsbeispiel 33 3-phasige Bedingungen mit Aliquat® 336 als PTC (Zugänglichkeit q = 1,38)**

100 mg nicht voraktivierte pll, 59 mg Phenyl-*E*-styrylsulfon sowie 11 mg Aliquat® 336 wurden in einer Mischung aus 0.8 ml Toluol und 200 µl NaOH (eingesetzt als 5 molare wässrige Lösung, entspricht 4,2 Äquivalenten) suspendiert. Anschließend wurden 125 µl $H_2O_2$ (eingesetzt als 30%ige wässrige Lösung, entspricht 5 Äquivalenten) zugesetzt. Diese Mischung wurde bei Raumtemperatur unter Rühren umgesetzt. Nach 2 h Reaktionszeit wurde die Reaktionsmischung mit 2 ml Ethylacetat verdünnt und anschließend zentrifugiert. Der Überstand wurde dann langsam in 2 ml Wasser eingetragen. Nach der Phasentrennung wurde die organische Phase über Natriumsulfat getrocknet und unter verringertem Druck eingeengt.

Die Ergebnisse des Beispiels 32 und des Vergleichsbeispiels 33 sind in der nachfolgenden Tabelle 8 zusammengefasst.

Tabelle 8

| Beispiel | Bedingungen | PTC | Reaktionszeit [h] | Umsatz [%] | ee [%] |
|---|---|---|---|---|---|
| 32 | erfindungsgemäß | $(Bu_4N)^+Br^-$ | 2 | 79 | 53 |
| VB33 | nicht erfindungsgemäß | Aliquat® 336 | 2 | 92 | 22 |

## Patentansprüche

**1.** Verfahren zur Epoxidierung von α,β-ungesättigten Enonen oder α,β-ungesättigten Sulfonen in Gegenwart

(1) einer wasserlöslichen Base,
(2) eines Oxidationsmittels,
(3) einer diastereomeren- und enantiomerenangereicherten Homo-Polyaminosäure als Katalysator,
(4) Wasser und
(5) eines mit Wasser nicht oder nur begrenzt mischbaren organischen Lösungsmittels,
**dadurch gekennzeichnet, dass** zusätzlich
(6) in Gegenwart eines Phasentransferkatalysators der Formel (I)

$$(R^1R^2R^3R^4A)^+X^- \quad (I)$$

gearbeitet wird, wobei

| | |
|---|---|
| A | für N oder P steht |
| $X^-$ | für ein anorganisches oder organisches Anion steht, |
| $R^1$, $R^2$, $R^3$ und $R^4$ | gleich oder verschieden sind und für Alkyl-, Aryl-, Aralkyl-, Cycloalkyl- oder Heteroaryl-Reste stehen, die durch einen oder mehrere, gleiche oder verschiedene Halogen-Reste substituiert sein können, oder aber jeweils zwei Reste unter Einbindung von A einen $C_4$-$C_6$-Cycloalkyl-Ring bilden können, |

und wobei

(i) die Summe der in den Resten $R^1$, $R^2$, $R^3$ und $R^4$ enthaltenen Kohlenstoff- und Heteroatome mindestens 13 beträgt und
(ii) die Zugänglichkeit q des Phasentransferkatalysators im Bereich von 0,6-1,3 liegt, wobei sich q durch folgende Formel ergibt:

$$q = \sum_{x=1}^{4} \left[ 1 / (\text{Summe der Kohlenstoff- und Heteroatome in } R^x) \right]$$

2. Verfahren nach Anspruch 1, **dadurch gekennzeichnet, dass** ein Phasentransferkatalysator eingesetzt wird, dessen Zugänglichkeit q im Bereich von 0,7-1,3 und bevorzugt im Bereich von 0,8-1,2 liegt.

3. Verfahren nach Anspruch 1 oder 2, **dadurch gekennzeichnet, dass** in der allgemeinen Formel (I) X für $F^-$, $Cl^-$, $Br^-$, $I^-$, $OH^-$, $NO_3^-$, $HSO_4^-$, $SO_4^-$, $CH_3COO^-$, $CF_3COO^-$, $C_2H_5COO^-$ , $C_3H_7COO^-$, $CF_3SO_3^-$ oder $C_4F_9SO_3^-$ steht.

4. Verfahren nach einem oder mehreren der Ansprüche 1-3, **dadurch gekennzeichnet, dass** solche Phasentransferkatalysatoren der allgemeinen Formel (I) eingesetzt werden, bei denen $R^1$, $R^2$, $R^3$ und $R^4$ gleich oder verschieden sind und für $C_1$-$C_{18}$-Alkyl, $C_6$-$C_{18}$-Aryl, $C_7$-$C_{19}$-Aralkyl, $C_5$-$C_7$-Cycloalkyl oder $C_3$-$C_{18}$-Heteroaryl stehen.

5. Verfahren nach Anspruch 1, **dadurch gekennzeichnet, dass** als Phasentransferkatalysator $((C_4H_9)_4N)^+Hal^-$, bevorzugt $((C_4H_9)_4N)^+Br^-$, $((C_4H_9)_4P)^+Hal^-$, bevorzugt $((C_4H_9)_4P)^+Br^-$, oder $((C_4H_9)_4N)^+HSO_4^-$ eingesetzt werden.

6. Verfahren nach einem oder mehreren der Ansprüche 1-5, **dadurch gekennzeichnet, dass** der Phasentransferkatalysator in einer Menge im Bereich von 0.1 - 20 mol%, bevorzugt im Bereich von 0,5-15 mol%, besonders bevorzugt im Bereich von 0,5-11 mol%, jeweils bezogen auf das eingesetzte α,β-ungesättigte Enon oder α,β-ungesättigte Sulfon, eingesetzt wird.

7. Verfahren nach einem oder mehreren der Ansprüche 1-6, **dadurch gekennzeichnet, dass** als α,β-ungesättigte Enone oder α,β-ungesättigte Sulfone Verbindungen der allgemeinen Formel (II)

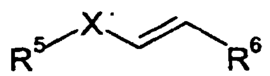

(II)

eingesetzt werden, worin

X für (C=O) oder ($SO_2$) steht und

$R^5$ und $R^6$ gleich oder verschieden sind und ($C_1$-$C_{18}$)-Alkyl, ($C_2$-$C_{18}$)-Alkenyl, ($C_2$-$C_{18}$)-Alkinyl, ($C_3$-$C_8$)-Cycloalkyl, ($C_6$-$C_{18}$)-Aryl, ($C_7$-$C_{19}$)-Aralkyl, ($C_1$-$C_{18}$)-Heteroaryl oder ($C_2C_{19}$)-Heteroaralkyl bedeuten, wobei die für $R^5$ und $R^6$ genannten Reste einfach oder mehrfach mit gleichen oder verschiedenen Resten Rest $R^7$, Halogen, $NO_2$, $NR^7R^8$, $PO_{0-3}R^7R^8$, $SO_{0-3}R^7$, $OR^7$, $CO_2R^7$, $CONHR^7$ oder $COR^7$ substituiert sein können und gegebenenfalls eine oder mehrere $CH_2$-Gruppen in den Resten $R^5$ und $R^6$ durch O, $SO_{0-2}$, $NR^7$ oder $PO_{0-2}R^7$ substituiert sein können,

wobei $R^7$ und $R^8$ gleich oder verschieden sind und H, ($C_1$-$C_{18}$)-Alkyl, ($C_2$-$C_{18}$)-Alkenyl, ($C_2$-$C_{18}$)-Alkinyl, ($C_3$-$C_8$)-Cycloalkyl, ($C_6$-$C_{18}$)-Aryl, ($C_1$-$C_{18}$)-Heteroaryl, ($C_1$-$C_8$)-Alkyl-($C_6$-$C_8$)-Aryl, ($C_1$-$C_8$)-Alkyl-($C_1$-$C_{18}$)-Heteroaryl, ($C_1$-$C_8$)-Alkyl-($C_3$-$C_8$)-Cycloalkyl bedeuten und diese Reste $R^7$ und $R^8$ einfach oder mehrfach mit gleichen oder verschiedenen Halogenresten substituiert sein können.

8. Verfahren nach einem oder mehreren der Ansprüche 1-7, **dadurch gekennzeichnet, dass** als α,β-ungesättigte Enone oder α,β-ungesättigte Sulfone Verbindungen der allgemeinen Formel (II) eingesetzt werden, in denen $R^5$ und $R^6$ gleich oder verschieden sind und ($C_1$-$C_{12}$)-Alkyl, ($C_2$-$C_{12}$)-Alkenyl, ($C_2$-$C_{12}$)-Alkinyl, ($C_5$-$C_8$)-Cycloalkyl, ($C_6$-$C_{12}$)-Aryl oder ($C_1$-$C_{12}$)-Heteroaryl bedeuten, wobei die zuvor genannten Reste einfach oder mehrfach mit gleichen oder verschiedenen Resten $R^7$, Halogen, $NO_2$, $NR^7R^8$, $PO_{0-3}R^7R^8$ oder $OR^7$ substituiert sein können und $R^7$ und $R^8$ die für die allgemeine Formel (II) angegebenen Bedeutungen besitzen.

9. Verfahren nach einem oder mehreren der Ansprüche 1-8, **dadurch gekennzeichnet, dass** als Substrate des erfindungsgemäßen Verfahrens α,β-ungesättigte Enone oder α,β-ungesättigte Sulfone der allgemeinen Formel (II) eingesetzt werden, in denen $R^5$ und $R^6$ gleich oder verschieden sind und ($C_1$-$C_{12}$)-Alkyl, ($C_2$-$C_{12}$)-Alkenyl,

($C_2$-$C_{12}$)-Alkinyl, ($C_5$-$C_8$)-Cycloakyl, ($C_6$-$C_{12}$)-Aryl oder ($C_1$-$C_{12}$)-Heteroaryl bedeuten, wobei die zuvor genannten Reste einfach oder mehrfach mit gleichen oder verschiedenen Resten $R^7$, Halogen, $NO_2$, $NR^7R^8$, $PO_{0-3}R^7R^8$ oder $OR^7$ substituiert sein können und $R^7$ und $R^8$ die für die allgemeine Formel (II) zuvor angegebene Bedeutungen besitzen, mit der Maßgabe, dass mindestens einer der Reste $R^5$ oder $R^6$ einen ($C_2$-$C_{12}$)-Alkenyl-, ($C_2$-$C_{12}$)-Alkinyl-, ($C_6$-$C_{12}$)-Aryl- oder ($C_1$-$C_{12}$)-Heteroarylrest darstellt.

**10.** Verfahren nach einem oder mehreren der Ansprüche 1 bis 9, **dadurch gekennzeichnet, dass** als Substrate des erfindungsgemäßen Verfahrens Verbindungen der allgemeinen Formel (III) eingesetzt werden,

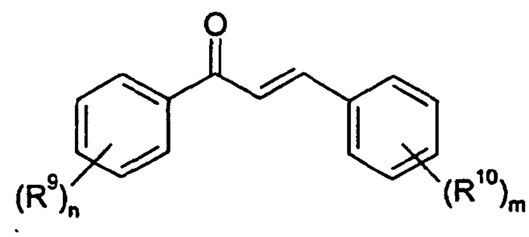

(III)

wobei

n und m gleich oder verschieden sind und für die Zahlen 0, 1, 2 oder 3 stehen, und

$R^9$ und $R^{10}$ gleich oder verschieden sind und $NR^7R^8$, $NO_2$, $OR^7$, ($C_1$-$C_{12}$)-Alkyl, ($C_2$-$C_{12}$)-Alkenyl, ($C_2$-$C_{12}$)-Alkinyl ($C_5$-$C_8$)-Cycloalkyl, ($C_6$-$C_{12}$)-Aryl oder ($C_1$-$C_{12}$)-Heteroaryl bedeuten, wobei diese Reste $R^9$ und $R^{10}$ einfach oder mehrfach mit gleichen oder verschiedenen Halogenresten substituiert sein können und $R^7$ und $R^8$ die zuvor für Formel (II) genannten Bedeutungen besitzen.

**11.** Verfahren nach einem oder mehreren der Ansprüche 1-10, **dadurch gekennzeichnet, dass** als diastereomeren- und enantiomerenangereicherten Homo-Polyaminosäuren solche aus der Gruppe Polyneopentylglycin, Polyleucin, Polyisoleucin, Polyvalin, Polyalanin und Polyphenylalanin verwendet werden.

**12.** Verfahren nach einem oder mehreren der Ansprüche 1-11, **dadurch gekennzeichnet, dass** die Kettenlänge der Polyaminosäuren im Bereich von 5 bis 100, vorzugsweise im Bereich von 7 bis 50 und insbesondere im Bereich von 10 bis 40 Aminosäure-Wiederholungseinheiten liegt.

**13.** Verfahren nach einem oder mehreren der Ansprüche 1-12, **dadurch gekennzeichnet, dass** die Homo-Polyaminosäuren vor ihrem Einsatz als Katalysator der Epoxidierung keiner separaten Voraktivierung mit Zwischenisolierung unterzogen werden und nicht auf einen anorganischen Träger aufgebracht werden.

**14.** Verfahren nach einem oder mehreren der Ansprüche 1-13, **dadurch gekennzeichnet, dass** die Homo-Polyaminosäure im Bereich von 0.0001 - 40 mol%, bevorzugt im Bereich von 0,001-20 mol%, besonders bevorzugt im Bereich von 0,01-15 mol%, und insbesondere im Bereich von 1 bis 15 mol-%, jeweils bezogen auf das eingesetzte $\alpha,\beta$-ungesättigte Enon oder $\alpha,\beta$-ungesättigte Sulfon, eingesetzt wird.

**15.** Verfahren nach einem oder mehreren der Ansprüche 1-14, **dadurch gekennzeichnet, dass** als Oxidationsmittel Peroxide, Persäuren oder anorganische Oxidationsmittel wie Natriumhypochlorit oder Natriumpercarbonat eingesetzt werden.

**16.** Verfahren nach einem oder mehreren der Ansprüche 1-15, **dadurch gekennzeichnet, dass** als Oxidationsmittel eine wässrige $H_2O_2$-Lösung eingesetzt wird.

**17.** Verfahren nach einem oder mehreren der Ansprüche 1-16, **dadurch gekennzeichnet, dass** das Oxidationsmittel in einer Menge von 1 - 40 Äquivalenten, bevorzugt von 1 - 10 Äquivalenten, besonders bevorzugt von 1-3 Äquivalenten und insbesondere 1,1 - 2,5 Äquivalenten eingesetzt wird.

**18.** Verfahren nach einem oder mehreren der Ansprüche 1-17, **dadurch gekennzeichnet, dass** als wasserlöslichen Base ein Alkalimetallhydroxid, bevorzugt NaOH, KOH oder LiOH eingesetzt wird.

**19.** Verfahren nach einem oder mehreren der Ansprüche 1-18, **dadurch gekennzeichnet, dass** die Base in einer Menge von 0,1-10 Äquivalenten, bevorzugt von 0,5-5 Äquivalenten und besonders bevorzugt von 0,8-2 Äquivalenten eingesetzt wird.

**20.** Verfahren nach einem oder mehreren der Ansprüche 1-19, **dadurch gekennzeichnet, dass** als organisches Lösungsmittel unsubstituierte oder substituierte aromatische Kohlenwasserstoffe, bevorzugt Toluol oder Xylol, aliphatische Kohlenwasserstoffe, bevorzugt Hexan, Halogenalkane, bevorzugt Chloroform oder Methylenchlorid, oder Ether, bevorzugt *tert.*-Butylmethylether und Diethylether eingesetzt werden.

**21.** Verfahren nach einem oder mehreren der Ansprüche 1-20, **dadurch gekennzeichnet, dass** die Reaktiontemperatur im Bereich von -10 bis +50 °C, bevorzugt im Bereich von 0 bis +40 °C und insbesondere bei +10 bis +30 °C liegt.

## Claims

**1.** Process for the epoxidation of α,β-unsaturated enones or α,β-unsaturated sulphones in the presence of

(1) a water-soluble base,
(2) an oxidant,
(3) a diastereomer- and enantiomer-enriched homo-polyamino acid as catalyst,
(4) water and
(5) an organic solvent which is immiscible or has only limited miscibility with water,
**characterized in that** additionally
(6) a phase-transfer catalyst of the formula (I)

$$(R^1R^2R^3R^4A)^+X^- \qquad (I)$$

is present, where

A is N or P,

$X^-$ is an inorganic or organic anion,

$R^1$, $R^2$, $R^3$ and $R^4$ are identical or different and are alkyl, aryl, aralkyl, cycloalkyl or heteroaryl radicals which may be substituted by one or more identical or different halogen radicals, or else two radicals in each case may form a $C_4$-$C_6$-cycloalkyl ring including A,

where

(i) the total of the carbon atoms and heteroatoms present in the radicals $R^1$, $R^2$, $R^3$ and $R^4$ is at least 13, and
(ii) the accessibility q of the phase-transfer catalyst is in the range 0.6-1.3, where q results from the following formula:

$$q = \sum_{x=1}^{4} \left[ 1 / (\text{total of the carbon atoms and heteroatoms in } R^x) \right]$$

**2.** Process according to Claim 1, **characterized in that** a phase-transfer catalyst whose accessibility q is in the range 0.7-1.3, and preferably in the range 0.8-1.2, is employed.

**3.** Process according to Claim 1, or 2, **characterized in that** X in the general formula (I) is $F^-$, $Cl^-$, $Br^-$, $I^-$, $OH^-$, $NO_3^-$, $HSO_4^-$, $SO_4^-$, $CH_3COO^-$, $CF_3COO^-$, $C_2H_5COO^-$, $C_3H_7COO^-$ , $CF_3SO_3^-$ or $C_4F_9SO_3^-$.

**4.** Process according to one or more of Claims 1-3, **characterized in that** phase-transfer catalysts of the general formula (I) in which $R^1$, $R^2$, $R^3$ and $R^4$ are identical or different and are $C_1$-$C_{18}$-alkyl, $C_6$-$C_{18}$-aryl, $C_7$-$C_{19}$-aralkyl, $C_5$-$C_7$-cycloalkyl or $C_3$-$C_{18}$-heteroaryl are employed.

**5.** Process according to Claim 1, **characterized in that** $((C_4H_9)_4N)^+Hal^-$, preferably $((C_4H_9)_4N)^+Br^-$, $((C_4H_9)_4P)^+Hal^-$, preferably $((C_4H_9)_4P)^+Br^-$, or $((C_4H_9)_4N)^+HSO_4^-$ are employed as phase-transfer catalyst.

**6.** Process according to one or more of Claims 1-5, **characterized in that** the phase-transfer catalyst is employed in an amount in the range 0.1-20 mol%, preferably in the range 0.5-15 mol%, particularly preferably in the range 0.5-11 mol%, in each case based on the α,β-unsaturated enone or α,β-unsaturated sulphone employed.

**7.** Process according to one or more of Claims 1-6, **characterized in that** the compounds employed as α,β-unsaturated enones or α,β-unsaturated sulphones have the general formula (II)

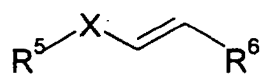

(II)

in which

X is (C=O) or ($SO_2$), and

$R^5$ and $R^6$ are identical or different and are ($C_1$-$C_{18}$)-alkyl, ($C_2$-$C_{18}$)-alkenyl, ($C_2$-$C_{18}$)-alkynyl, ($C_3$-$C_8$)-cycloalkyl, ($C_6$-$C_{18}$)-aryl, ($C_7$-$C_{19}$)-aralkyl, ($C_1$-$C_{18}$)-heteroaryl or ($C_2$-$C_{19}$)-heteroaralkyl,

where the radicals mentioned for $R^5$ and $R^6$ may be substituted once or more than once by identical or different radicals $R^7$, halogen, $NO_2$, $NR^7R^8$, $PO_{0-3}R^7R^8$, $SO_{0-3}R^7$, $OR^7$, $CO_2R^7$, $CONHR^7$ or $COR^7$, and optionally one or more $CH_2$ groups in the radicals $R^5$ and $R^6$ are replaced by O, $SO_{0-2}$, $NR^7$ or $PO_{0-2}R^7$,

where $R^7$ and $R^8$ are identical or different and are H, ($C_1$-$C_{18}$)-alkyl, ($C_2$-$C_{18}$)-alkenyl, ($C_2$-$C_{18}$)-alkynyl, ($C_3$-$C_8$)-cycloalkyl, ($C_6$-$C_{18}$)-aryl, ($C_1$-$C_{18}$)-heteroaryl, ($C_1$-$C_8$)-alkyl-($C_6$-$C_8$)-aryl, ($C_1$-$C_8$)-alkyl-($C_1$-$C_{18}$)-heteroaryl, ($C_1$-$C_8$)-alkyl-($C_3$-$C_8$)-cycloalkyl, and these radicals $R^7$ and $R^8$ may be substituted once or more than once by identical or different halogen radicals.

**8.** Process according to one or more of Claims 1-7, **characterized in that** the compounds employed as α,β-unsaturated enones or α,β-unsaturated sulphones have the general formula (II) in which $R^5$ and $R^6$ are identical or different and are ($C_1$-$C_{12}$)-alkyl, ($C_2$-$C_{12}$)-alkenyl, ($C_2$-$C_{12}$)-alkynyl, ($C_5$-$C_8$)-cycloalkyl, ($C_6$-$C_{12}$)-aryl or ($C_1$-$C_{12}$)-heteroaryl, where the aforementioned radicals may be substituted once or more than once by identical or different radicals $R^7$, halogen, $NO_2$, $NR^7R^8$, $PO_{0-3}R^7R^8$ or $OR^7$, and $R^7$ and $R^8$ have the meanings indicated above for the general formula (II).

**9.** Process according to one or more of Claims 1-8, **characterized in that** the substrates employed in the process according to the invention are α,β-unsaturated enones or α,β-unsaturated sulphones of the general formula (II) in which $R^5$ and $R^6$ are identical or different and are ($C_1$-$C_{12}$)-alkyl, ($C_2$-$C_{12}$)-alkenyl, ($C_2$-$C_{12}$)-alkynyl, ($C_5$-$C_8$)-cycloalkyl, ($C_6$-$C_{12}$)-aryl or ($C_1$-$C_{12}$)-heteroaryl, where the aforementioned radicals may be substituted once or more than once by identical or different radicals $R^7$, halogen, $NO_2$, $NR^7R^8$, $PO_{0-3}R^7R^8$ or $OR^7$, and $R^7$ and $R^8$ have the meanings indicated above for the general formula (II), with the proviso that at least one of the radicals $R^5$ or $R^6$ is a ($C_2$-$C_{12}$)-alkenyl, ($C_2$-$C_{12}$)-alkynyl, ($C_6$-$C_{12}$)-aryl- or ($C_1$-$C_{12}$)-heteroaryl radical.

**10.** Process according to one or more of Claims 1 to 9, **characterized in that** the substrates employed in the process according to the invention are compounds of the general formula (III)

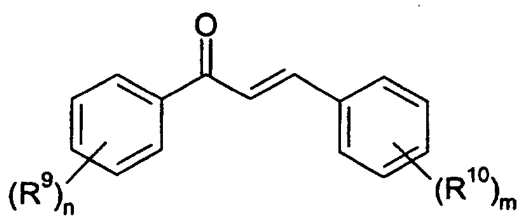

(III)

where

n and m are identical or different and are the numbers 0, 1, 2 or 3,

and

$R^9$ and $R^{10}$ are identical or different and are $NR^7R^8$, $NO_2$, $OR^7$, ($C_1$-$C_{12}$)-alkyl, ($C_2$-$C_{12}$)-alkenyl, ($C_2$-$C_{12}$)-alkynyl, ($C_5$-$C_8$)-cycloalkyl, ($C_6$-$C_{12}$)-aryl or ($C_1$-$C_{12}$)-heteroaryl where these radicals $R^9$ and $R^{10}$ may be substituted once or more than once by identical or different halogen radicals, and $R^7$ and $R^8$ have the meanings mentioned previously for formula (II).

**11.** Process according to one or more of Claims 1-10, **characterized in that** the diastereomer- and enantiomer-enriched homo-polyamino acids used are those from the group of polyneopentylglycine, polyleucine, polyisoleucine, polyvaline, polyalanine and polyphenylalanine.

**12.** Process according to one or more of Claims 1-11, **characterized in that** the chain length of the polyamino acids is in the range from 5 to 100, preferably in the range from 7 to 50, and in particular in the range from 10 to 40, amino acid repeating units.

**13.** Process according to one or more of Claims 1-12, **characterized in that** the homo-polyamino acids are not subjected before their use as catalyst of the epoxidation to any separate preactivation with intermediate isolation and are not applied to an inorganic support.

**14.** Process according to one or more of Claims 1-13, **characterized in that** the homo-polyamino acids are employed in the range 0.0001-40 mol%, preferably in the range 0.001-20 mol%, particularly preferably in the range 0.01-15 mol% and in particular in the range 1 to 15 mol%, in each case based on the $\alpha,\beta$-unsaturated enone or $\alpha,\beta$-unsaturated sulphone employed.

**15.** Process according to one or more of Claims 1-14, **characterized in that** peroxides, peracids or inorganic oxidants such as sodium hypochlorite or sodium percarbonate are employed as oxidants.

**16.** Process according to one or more of Claims 1-15, **characterized in that** an aqueous $H_2O_2$ solution is employed as oxidant.

**17.** Process according to one or more of Claims 1-16, **characterized in that** the oxidant is employed in an amount of 1-40 equivalents, preferably of 1-10 equivalents, particularly preferably of 1-3 equivalents and in particular 1.1-2.5 equivalents.

**18.** Process according to one or more of Claims 1-17, **characterized in that** an alkali metal hydroxide, preferably NaOH, KOH or LiOH, is employed as water-soluble base.

**19.** Process according to one or more of Claims 1-18, **characterized in that** the base is employed in an amount of 0.1-10 equivalents, preferably of 0.5-5 equivalents and particularly preferably of 0.8-2 equivalents.

**20.** Process according to one or more of Claims 1-19, **characterized in that** unsubstituted or substituted aromatic hydrocarbons, preferably toluene or xylene, aliphatic hydrocarbons, preferably hexane, haloalkanes, preferably chloroform or methylene chloride, or ethers, preferably tert-butyl methyl ether and diethyl ether, are employed as organic solvent.

21. Process according to one or more of Claims 1-20, **characterized in that** the reaction temperature is in the range from -10 to +50°C, preferably in the range from 0 to +40°C and in particular at +10 to +30°C.

## Revendications

1. Procédé pour l'époxydation d'énones α,β-insaturées ou de sulfones α,β-insaturées en présence

   (1) d'une base hydrosoluble,
   (2) d'un oxydant,
   (3) d'un homopolyaminoacide diastéréoisomériquement et énantiomériquement enrichi comme catalyseur,
   (4) d'eau et
   (5) d'un solvant organique immiscible à l'eau ou miscible à l'eau de manière limitée seulement
   **caractérisé en ce que** l'on opère en outre
   (6) en présence d'un catalyseur de transfert de phases de formule (I)

$$(R^1R^2R^3R^4)^+X^- \quad (I)$$

   où

   | | |
   |---|---|
   | A | représente N ou P, |
   | $X^-$ | représente un anion inorganique ou organique |
   | $R^1$,$R^2$,$R^3$ et $R^4$ | sont identiques ou différents et représentent des groupements alkyle, aryle, aralkyle, cycloalkyle ou hétéroaryle qui peuvent être substitués par un ou plusieurs groupements halogènes identiques ou différents, ou bien encore à chaque fois deux groupements peuvent former un cycle $C_4$-$C_6$-cycloalkyle avec inclusion de A, |

   où

   (i) la somme des atomes de carbone et des hétéroatomes contenus dans les groupements $R^1$, $R^2$, $R^3$ et $R^4$ est d'au moins 13 et
   (ii) l'accessibilité q du catalyseur de transfert de phases est située dans le domaine de 0,6-1,3, où q est donné par la formule suivante

$$q = \sum_{x=1}^{4} 1/(\text{somme des atomes de carbone et des hétéroatomes dans } R^x)]$$

2. Procédé selon la revendication 1 **caractérisé en ce que** l'on utilise un catalyseur de transfert de phases dont l'accessibilité q est située dans le domaine de 0,7-1,3 et de préférence dans le domaine de 0,8-1,2.

3. Procédé selon la revendication 1 ou 2 **caractérisé en ce que**, dans la formule générale (I), $X^-$ représente de préférence $F^-$, $Cl^-$, $Br^-$, $I^-$, $OH^-$, $NO_3^-$, $HSO_4^-$, $SO_4^-$, $CH_3COO^-$, $CF_3COO^-$, $C_2H_5COO^-$, $C_3H_7COO^-$, $CF_3SO_3^-$ ou $C_4F_9SO_3^-$.

4. Procédé selon une ou plusieurs des revendications 1-3 **caractérisé en ce que** l'on utilise des catalyseurs de transfert de phases de formule générale (I) dans lesquels $R^1$, $R^2$, $R^3$ et $R^4$ sont identiques ou différents et représentent $C_1$-$C_{18}$-alkyle, $C_6$-$C_{18}$-aryle, $C_7$-$C_{19}$-aralkyle, $C_5$-$C_7$-cycloalkyle ou $C_3$-$C_{18}$-hétéroaryle.

5. Procédé selon la revendication 1 **caractérisé en ce que** l'on utilise comme catalyseur de transfert de phases $((C_4H_9)_4N)^+Hal^-$, de préférence $((C_4H_9)_4N)^+Br^-$, $((C_4H_9)_4P)^+Hal^-$, de préférence $((C_4H_9)_4P)^+Br^-$ ou $((C_4H_9)_4N)^+HSO_4^-$.

6. Procédé selon une ou plusieurs des revendications 1-5 **caractérisé en ce que** le catalyseur de transfert de phases

est utilisé en une quantité dans le domaine de 0,1-20 mol %, de préférence dans le domaine de 0,5-15 mol %, de manière particulièrement préférée dans le domaine de 0,5-11 mol %, dans chaque cas par rapport à l'énone $\alpha,\beta$-insaturée ou à la sulfone $\alpha,\beta$-insaturée utilisée.

**7.** Procédé selon une ou plusieurs des revendications 1-6 **caractérisé en ce que** l'on utilise comme énones $\alpha,\beta$-insaturées ou sulfones $\alpha,\beta$-insaturées des composés de formule générale (II)

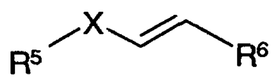

(II)

où

X représente (C=O) ou ($SO_2$) et

$R^5$ et $R^6$ sont identiques ou différents et représentent ($C_1$-$C_{18}$)-alkyle, ($C_2$-$C_{18}$)-alcényle, ($C_2$-$C_{18}$)-alcynyle, ($C_3$-$C_8$)-cycloalkyle, ($C_6$-$C_{18}$)-aryle, ($C_7$-$C_{19}$)-aralkyle, ($C_1$-$C_{18}$)-hétéroaryle ou ($C_2$-$C_{19}$)-hétéroaralkyle,
où les groupements cités pour $R^5$ et $R^6$ peuvent être substitués une fois ou plusieurs fois par des groupements identiques ou différents ($R^7$, halogène, $NO_2$, $NR^7R^8$, $NR^7R^8$, $PO_{0-3}R^7R^8$, $SO_{0-3}R^7$, $OR^7$, $CO_2R^7$, $CONHR^7$ ou $COR^7$ et éventuellement un ou plusieurs groupes $CH_2$ dans les groupements $R^5$ et $R^6$ sont substitués par O, $SO_{0-2}$, $NR^7$ ou $PO_{0-2}R^7$,
où $R^7$ et $R^8$ sont identiques ou différents et représentent H, ($C_1$-$C_{18}$)-alkyle, ($C_2$-$C_{18}$)-alcényle, ($C_2$-$C_{18}$)-alcynyle, ($C_3$-$C_8$)-cycloalkyle, ($C_6$-$C_{18}$)-aryle, ($C_1$-$C_{18}$)-hétéroaryle, ($C_1$-$C_8$)-alkyl-($C_6$-$C_8$)-aryle, ($C_1$-$C_8$)-alkyl-($C_1$-$C_{19}$)-hétéroaryle, ($C_1$-$C_8$)-alkyl-($C_3$-$C_8$)-cycloalkyle et ces groupements $R^7$ et $R^8$ peuvent être substitués une fois ou plusieurs fois par des groupements halogènes identiques ou différents.

**8.** Procédé selon une ou plusieurs des revendications 1-7 **caractérisé en ce que** l'on utilise comme énones $\alpha,\beta$-insaturées ou sulfones $\alpha,\beta$-insaturées des composés de formule générale (II) dans lesquels $R^5$ et $R^6$ sont identiques ou différents et représentent ($C_1$-$C_{12}$)-alkyle, ($C_2$-$C_{12}$)-alcényle, ($C_2$-$C_{12}$)-alcynyle, ($C_5$-$C_8$)-cycloalkyle, ($C_6$-$C_{12}$)-aryle ou ($C_1$-$C_{12}$)-hétéroaryle, où les groupements cités précédemment peuvent être substitués une fois ou plusieurs fois par des groupements identiques ou différents $R^7$, halogène, $NO_2$, $NR^7R^8$, $PO_{0-3}R^7R^8$ ou $OR^7$ et $R^7$ et $R^8$ possèdent les significations indiquées pour la formule générale (II).

**9.** Procédé selon une ou plusieurs des revendications 1-8 **caractérisé en ce que** l'on utilise comme substrats du procédé selon l'invention des énones $\alpha,\beta$-insaturées ou des sulfones $\alpha,\beta$-insaturées de formule générale (II), dans lesquelles $R^5$ et $R^6$ sont identiques ou différents et représentent ($C_1$-$C_{12}$)-alkyle, ($C_2$-$C_{12}$)-alcényle, ($C_2$-$C_{12}$)-alcynyle, ($C_5$-$C_8$)-cycloalkyle, ($C_6$-$C_{12}$)-aryle ou ($C_1$-$C_{12}$)-hétéroaryle, où les groupements cités précédemment peuvent être substitués une fois ou plusieurs fois par des groupements identiques ou différents $R^7$, halogène, $NO_2$, $NR^7R^8$, $PO_{0-3}R^7R^8$ ou $OR^7$ et $R^7$ et $R^8$ possèdent les significations indiquées précédemment pour la formule générale (II),
avec la condition qu'au moins l'un des groupements $R^5$ ou $R^6$ représente un groupement ($C_2$-$C_{12}$)-alcényle, ($C_2$-$C_{12}$)-alcynyle, ($C_6$-$C_{12}$)-aryle ou ($C_1$-$C_{12}$)-hétéroaryle.

**10.** Procédé selon une ou plusieurs des revendications 1 à 9 **caractérisé en ce que** l'on utilise comme substrats du procédé selon l'invention des composés de formule générale (III) :

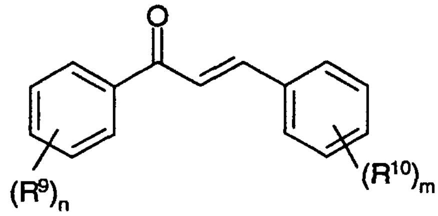

(III)

où

n et m sont identiques ou différents et représentent les nombres 0, 1, 2 ou 3,

et

$R^9$ et $R^{10}$ sont identiques ou différents et représentent $NR^7R^8$, $NO_2$, $OR^7$, ($C_1$-$C_{12}$)-alkyle, ($C_2$-$C_{12}$)-alcényle, ($C_2$-$C_{12}$)-alcynyle, ($C_5$-$C_8$)-cycloalkyle, ($C_6$-$C_{12}$)-aryle ou ($C_1$-$C_{12}$)-hétéroaryle, où ces groupements $R^9$ et $R^{10}$ peuvent être substitués une fois ou plusieurs fois par des groupements halogènes identiques ou différents et $R^7$ et $R^8$ possèdent les significations citées précédemment pour la formule (II).

**11.** Procédé selon une ou plusieurs des revendications 1-10 **caractérisé en ce que** comme homopolyaminoacides diastéréoisomériquement et énantiomériquement enrichis on utilise ceux du groupe de la polynéopentylglycine, de la polyleucine, de la polyisoleucine, de la polyvaline et de la polyalanine et de la polyphénylalanine.

**12.** Procédé selon une ou plusieurs des revendications 1-11 **caractérisé en ce que** la longueur de chaîne des polyaminoacides est située dans le domaine de 5 à 100, de préférence dans le domaine de 7 à 50 et en particulier dans le domaine de 10 à 40 unités répétées d'aminoacide.

**13.** Procédé selon une ou plusieurs des revendications 1-12 **caractérisé en ce que** les homopolyaminoacides, avant leur utilisation comme catalyseur d'époxydation, ne sont soumis à aucune préactivation séparée avec isolement intermédiaire et ne sont pas appliqués sur un support inorganique.

**14.** Procédé selon une ou plusieurs des revendications 1-13 **caractérisé en ce que** l'homopolyaminoacide est utilisé dans le domaine de 0,0001-40 mol %, de préférence dans le domaine de 0,001-20 mol %, de manière particulièrement préférée dans le domaine de 0,01-15 mol %, et en particulier dans le domaine de 1 à 15 mol %, dans chaque cas par rapport à l'énone $\alpha,\beta$-insaturée ou à la sulfone $\alpha,\beta$-insaturée utilisée.

**15.** Procédé selon une ou plusieurs des revendications 1-14 **caractérisé en ce que** des peroxydes, des peracides ou des oxydants inorganiques comme l'hypochlorite de sodium ou le percarbonate de sodium sont utilisés comme oxydants.

**16.** Procédé selon une ou plusieurs des revendications 1-15 **caractérisé en ce qu'**une solution aqueuse de $H_2O_2$ est utilisée comme oxydant.

**17.** Procédé selon une ou plusieurs des revendications 1-16 **caractérisé en ce que** l'oxydant est utilisé en une quantité de 1-40 équivalents, de préférence de 1-10 équivalents, de manière particulièrement préférée de 1-3 équivalents et en particulier de 1,1-2,5 équivalents.

**18.** Procédé selon une ou plusieurs des revendications 1-17 **caractérisé en ce qu'**un hydroxyde de métal alcalin, de préférence NaOH, KOH ou LiOH est utilisé de préférence comme base hydrosoluble.

**19.** Procédé selon une ou plusieurs des revendications 1-18 **caractérisé en ce que** la base est utilisée en une quantité de 0,1-10 équivalents, de préférence de 0,5-5 équivalents et de manière particulièrement préférée de 0,8-2 équivalents.

**20.** Procédé selon une ou plusieurs de revendications 1-19 **caractérisé en ce que** l'on utilise comme solvant organique des hydrocarbures aromatiques non substitués ou substitués, de préférence le toluène ou le xylène, des hydrocarbures aliphatiques, de préférence l'hexane, des halogénoalcanes, de préférence le chloroforme ou le chlorure de méthylène, ou des éthers, de préférence le *tert.*-butylméthyléther et le diéthyléther.

**21.** Procédé selon une ou plusieurs des revendications 1-20 **caractérisé en ce que** la température de réaction est située dans le domaine de -10 à +50°C, de préférence dans le domaine de 0 à +40°C et en particulier à +10 à +30°C.